# EUROPEAN PATENT APPLICATION

(11) **EP 4 541 817 A2**
(43) Date of publication of application: **23.04.2025**
(21) Application number: 24199745.1
(22) Date of filing: 11.09.2024
(51) Int. Cl.: C07K 16/28, C07K 16/30, A61K 40/31, C07K 14/725

(54) **NUCLEIC ACID MOLECULE ENCODING CAR AND VECTOR INCLUDING THE SAME, IMMUNE CELL COMPRISING CAR AND PHARMACEUTICAL COMPOSITION INCLUDING THE CELL, METHOD FOR IMPROVING CYTOTOXIC ACTIVITY, AND METHOD FOR PRODUCING IMMUNE CELL COMPRISING CAR**

(30) Priority: 12.09.2023 JP 2023147779
(71) Applicant: Mie University, Tsu-shi, Mie 514-8507 (JP); SYSMEX CORPORATION, Kobe-shi Hyogo 651-0073 (JP)
(72) Inventor: AKAHORI, Yasushi, Tsu-shi, Mie 514-8507 (JP); MAETA, Shingo, Kobe-shi, Hyogo 651-0073 (JP); FUKUNAGA, Atsushi, Kobe-shi, Hyogo 651-0073 (JP); EGASHIRA, Yuriko, Kobe-shi, Hyogo 651-0073 (JP)
(74) Representative: Hoffmann Eitle

(57) **Abstract**

Disclosed is a nucleic acid molecule having a nucleotide sequence encoding a chimeric antigen receptor, wherein
the nucleic acid molecule comprises a segment encoding an extracellular domain, a segment encoding a transmembrane domain, and a segment encoding an intracellular domain,
the segment encoding the extracellular domain comprises a nucleotide sequence encoding an antigen binding region comprising a light chain variable region and a heavy chain variable region,
at least three codons in a nucleotide sequence encoding framework region 3 of the light chain variable region as defined by the Chothia method are codons encoding acidic amino acid residues, and
the at least three codons comprise at least three selected from the group consisting of a codon encoding an amino acid residue at position 60, a codon encoding an amino acid residue at position 63, a codon encoding an amino acid residue at position 65, a codon encoding an amino acid residue at position 67, a codon encoding an amino acid residue at position 70, a codon encoding an amino acid residue at position 72, a codon encoding an amino acid residue at position 74, and a codon encoding an amino acid residue at position 76 in the light chain variable region.

## Description

### FIELD OF THE INVENTION

The present invention relates to a nucleic acid molecule having a nucleotide sequence encoding a chimeric antigen receptor (hereinafter, also referred to as "CAR"). The present invention relates to a vector including the nucleic acid molecule. The present invention relates to an immune cell including the CAR. The present invention relates to a pharmaceutical composition for treating a malignant tumor including the immune cell including the CAR. The present invention relates to a method for improving the cytotoxic activity of an immune cell including the CAR. The present invention relates to a method for producing an immune cell including the CAR.

### BACKGROUND

The CAR is a receptor protein produced by genetically engineering fusion of an extracellular domain including an antigen binding region, a transmembrane domain, and an intracellular domain that transmits an activation signal of an immune cell. For example, as described in US Patent No. 7,741,465, a single-chain antibody that binds to an antigen expressed in a tumor cell is used for the antigen binding region of the CAR. In recent years, cancer immunotherapy has attracted attention in which a gene encoding a CAR is introduced into an immune cell, and an immune cell expressing the CAR on the cell surface is transplanted to a patient to treat cancer. When the immune cell expressing the CAR recognizes an antigen of a tumor cell in vivo, the immune cell is activated to express cytotoxic molecules such as Fas ligand, perforin, and granzyme and cytokines, and exerts an antitumor effect.

### SUMMARY OF THE INVENTION

An object of the present invention is to provide an improved CAR capable of improving the cytotoxic activity of the immune cell expressing the CAR. An object of the present invention is to provide a nucleic acid molecule encoding such a CAR and a vector including the nucleic acid molecule. An object of the present invention is to provide an immune cell including such a CAR, a pharmaceutical composition including the immune cell, a method for improving the cytotoxic activity of the immune cell, and a method for producing the immune cell.

The present inventors have found that when a predetermined amino acid residue in framework region 3 of the light chain variable region of the antigen binding region of a CAR is an acidic amino acid residue, cytotoxic activity by the immune cell including the CAR can be improved, thereby completing the present invention. Therefore, the following inventions [1] to [20] are provided. Hereinafter, the framework region 3 is also referred to as "FR3".
[1] A nucleic acid molecule comprising a nucleotide sequence encoding a CAR, wherein the nucleic acid molecule includes a segment encoding an extracellular domain, a segment encoding a transmembrane domain, and a segment encoding an intracellular domain, the segment encoding the extracellular domain includes a nucleotide sequence encoding an antigen binding region comprising a light chain variable region and a heavy chain variable region, at least three codons in a nucleotide sequence encoding FR3 of the light chain variable region as defined by the Chothia method are codons encoding acidic amino acid residues, the at least three codons include at least three selected from the group consisting of a codon encoding an amino acid residue at position 60, a codon encoding an amino acid residue at position 63, a codon encoding an amino acid residue at position 65, a codon encoding an amino acid residue at position 67, a codon encoding an amino acid residue at position 70, a codon encoding an amino acid residue at position 72, a codon encoding an amino acid residue at position 74, and a codon encoding an amino acid residue at position 76 in the light chain variable region.
[2] The nucleic acid molecule according to [1], wherein 3 or more and 5 or less codons selected from the group consisting of a codon encoding an amino acid residue at position 60, a codon encoding an amino acid residue at position 63, a codon encoding an amino acid residue at position 65, a codon encoding an amino acid residue at position 67, a codon encoding an amino acid residue at position 70, a codon encoding an amino acid residue at position 72, a codon encoding an amino acid residue at position 74, and a codon encoding an amino acid residue at position 76 in the light chain variable region are codons encoding an acidic amino acid residue.
[3] The nucleic acid molecule according to [1] or [2], wherein the antigen binding region includes a single-chain antibody, and the single-chain antibody is a single-chain antibody that binds to a complex of a MAGE-A4 derived peptide and HLA-A2, CD19, BCMA, or CEA.
[4] The nucleic acid molecule according to any one of [1] to [3], wherein the transmembrane domain includes a transmembrane region of any one protein selected from the group consisting of an α chain of a T cell receptor, a β chain of a T cell receptor, CD3ε, CD3ζ, CD4, CD5, CD8, CD9, CD16, CD22, CD28, CD33, CD37, CD45, CD64, CD80, CD86, CD134, CD154, 4-1BB, ICOS, and GITR.
[5] The nucleic acid molecule according to any one of [1] to [4], wherein the intracellular domain includes a signaling domain of at least one protein selected from the group consisting of CD3ζ, CD3γ, CD3δ, CD3ε, CD5, CD22, CD66d, CD79a, CD79b, FcRγ, and FcRβ.
[6] The nucleic acid molecule according to [5], wherein the segment encoding the intracellular domain further includes a nucleotide sequence encoding a co-stimulatory domain, and the co-stimulatory domain is a co-stimulatory domain of at least one protein selected from the group consisting of 4-1BB, CD28, GITR, CD2, CD5, CD8, CD9, CD16, CD22, CD33, CD37, CD64, CD80, CD86, CD134, CD154 and ICOS.
[7] The nucleic acid molecule according to any one of [1] to [6], further comprising a segment encoding a hinge domain between the nucleotide sequence encoding the antigen binding region and the segment encoding the transmembrane domain.
[8] The nucleic acid molecule according to any one of [1] to [7], wherein the nucleic acid molecule is DNA or RNA.
[9] A vector including the nucleic acid molecule according to any one of [1] to [8].
[10] A CAR receptor comprising an extracellular domain, a transmembrane domain and an intracellular domain, wherein the extracellular domain includes an antigen binding region comprising a light chain variable region and a heavy chain variable region, at least three amino acid residues of FR3 of the light chain variable region as defined by the Chothia method are acidic amino acid residues, the at least three amino acid residues includes at least three selected from the group consisting of an amino acid residue at position 60, an amino acid residue at position 63, an amino acid residue at position 65, an amino acid residue at position 67, an amino acid residue at position 70, an amino acid residue at position 72, an amino acid residue at position 74, and an amino acid residue at position 76 in the light chain variable region.
[11] The CAR according to [10], wherein 3 or more and 5 or less amino acid residues selected from the group consisting of an amino acid residue at position 60, an amino acid residue at position 63, an amino acid residue at position 65, an amino acid residue at position 67, an amino acid residue at position 70, an amino acid residue at position 72, an amino acid residue at position 74, and an amino acid residue at position 76 in the light chain variable region are acidic amino acid residues.
[12] The CAR according to [10] or [11], wherein the antigen binding region includes a single-chain antibody, and the single-chain antibody is a single-chain antibody that binds to a complex of a MAGE-A4 derived peptide and HLA-A2, CD19, BCMA, or CEA.
[13] The CAR according to any one of [10] to [12], wherein the transmembrane domain includes a transmembrane region of any one protein selected from the group consisting of an α chain of a T cell receptor, a β chain of a T cell receptor, CD3ε, CD3ζ, CD4, CD5, CD8, CD9, CD16, CD22, CD28, CD33, CD37, CD45, CD64, CD80, CD86, CD134, CD154, 4-1BB, ICOS, and GITR.
[14] The CAR according to any one of [10] to [13], wherein the intracellular domain includes a signaling domain of at least one protein selected from the group consisting of CD3ζ, CD3γ, CD3δ, CD3ε, CD5, CD22, CD66d, CD79a, CD79b, FcRy, and FcRβ.
[15] The CAR of [14], wherein the intracellular domain further includes a co-stimulatory domain, the co-stimulatory domain is a co-stimulatory domain of at least one protein selected from the group consisting of 4-1BB, CD28, GITR, CD2, CD5, CD8, CD9, CD16, CD22, CD33, CD37, CD64, CD80, CD86, CD134, CD154 and ICOS.
[16] The CAR of any one of [10] to [15], further comprising a hinge domain between the antigen binding region and the transmembrane domain.
[17] An immune cell including the chimeric antigen receptor according to any one of [10] to [16].
[18] A pharmaceutical composition for treating a malignant tumor, including the immune cell according to [17].
[19] A method for improving cytotoxic activity of an immune cell comprising a CAR, wherein the CAR includes an extracellular domain, a transmembrane domain and an intracellular domain, and the extracellular domain includes an antigen binding region comprising a light chain variable region and a heavy chain variable region, the method comprising changing at least three amino acid residues of FR3 of the light chain variable region defined by the Chothia method to acidic amino acid residues to improve cytotoxic activity of an immune cell including the CAR as compared to the CAR before changing the at least three amino acid residues to acidic amino acid residues.
[20] A method for producing an immune cell including the CAR, including introducing the nucleic acid molecule according to any one of [1] to [8] or the vector according to [9] into an immune cell, and expressing the CAR in the immune cell.

According to the present invention, an immune cell including the CAR having an improved cytotoxic activity can be obtained.

### BRIEF DESCRIPTION OF THE DRAWINGS

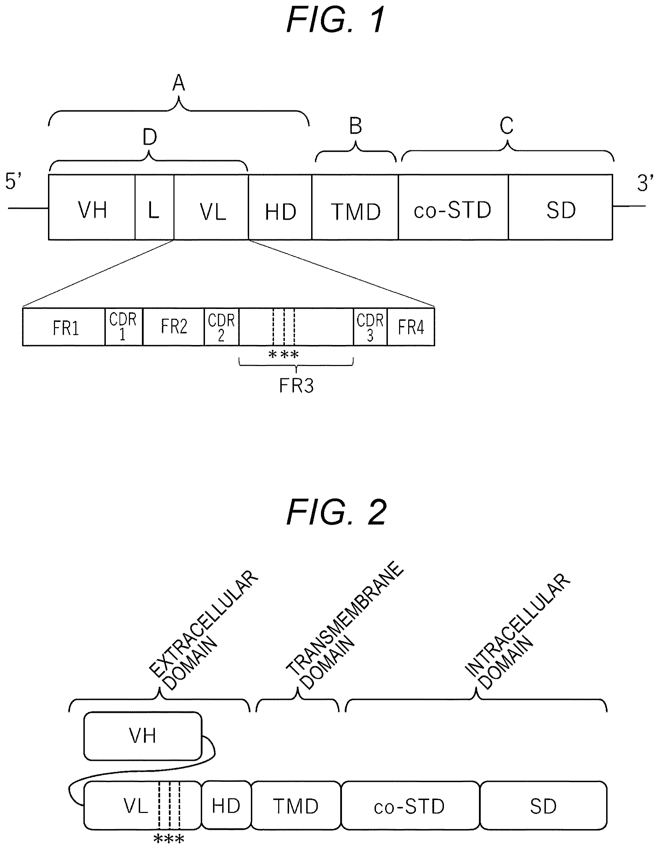

Fig. 1 is a diagram schematically showing an example of a nucleic acid molecule of the present embodiment. Also shown is an enlarged diagram of the segment encoding the light chain variable region (VL) in a nucleic acid molecule. In the figure, A denotes a segment encoding the extracellular domain, B denotes a segment encoding the transmembrane domain, and C denotes a segment encoding the intracellular domain. D denotes a segment encoding an antigen binding region included in the extracellular domain. In these segments, VH denotes a segment encoding a heavy chain variable region, L denotes a segment encoding a linker, HD denotes a segment encoding a hinge domain, TMD denotes a segment encoding a transmembrane domain, co-STD denotes a segment encoding a co-stimulatory domain, and SD denotes a segment encoding a signaling domain. In the enlarged diagram of the VL, dashed lines marked with * indicate codons encoding acidic amino acid residues. In this figure, in the segment encoding FR3, there are three codons encoding acidic amino acid residues, but the present invention is not limited thereto.
Fig. 2 is a diagram schematically showing an example of a CAR of the present embodiment. In the figure, VH denotes a heavy chain variable region, VL denotes a light chain variable region, HD denotes a hinge domain, TMD denotes a transmembrane domain, co-STD denotes a co-stimulatory domain, and SD denotes a signaling domain. The curve connecting VH and VL indicates the linker. In VL, the dashed line marked with * indicates acidic amino acid residues. In this figure, there are three acidic amino acid residues in FR3, but the present invention is not limited thereto.
Fig. 3 is a diagram schematically showing a nucleic acid molecule encoding the CAR of Examples 1 to 4.
Fig. 4 is a diagram schematically showing a nucleic acid molecule encoding the CAR of Example 5.
Fig. 5 is a diagram schematically showing a nucleic acid molecule encoding the CARs of Examples 6 and 7.

### DETAILED DESCRIPTION OF THE PREFERRED EMBODIMENTS

### 1. Nucleic acid molecule

The nucleic acid molecule of the present embodiment has a nucleotide sequence encoding a CAR. In the present specification, the term "nucleotide sequence" is synonymous with "base sequence" and "nucleic acid sequence". The nucleotide sequence refers to a one-dimensional arrangement (order) of nucleotides in a nucleic acid molecule. A nucleic acid molecule encoding a polypeptide has a nucleotide sequence encoding the polypeptide. In the present specification, the expression "having a nucleotide sequence" means both composed of the nucleotide sequence and including the nucleotide sequence. As used herein, the term "polypeptide" includes a protein molecule, a part of region within a protein molecule, and a fragment of a protein molecule. In the present specification, a part of region in a nucleic acid molecule may be referred to as a "segment", and a part of region in a protein molecule may be referred to as a "domain".

As exemplified in Fig. 1, the nucleic acid molecule of the present embodiment includes, in order from the 5' side, a segment encoding an extracellular domain, a segment encoding a transmembrane domain, and a segment encoding an intracellular domain. The segment encoding the extracellular domain includes a nucleotide sequence encoding an antigen binding region including a light chain variable region and a heavy chain variable region. In the present specification, the "antigen binding region including a light chain variable region and a heavy chain variable region" refers to a domain including at least one light chain variable region and at least one heavy chain variable region or capable of binding to a predetermined antigen via these regions. In the antigen binding region, one heavy chain variable region and one light chain variable region paired therewith constitute one antigen binding site. When the antigen binding region includes a plurality of heavy chain variable regions and a plurality of light chain variable regions, a plurality of antigen binding sites can be constituted in the antigen binding region.

Examples of the antigen binding region include a single-chain antibody. Hereinafter, the single-chain antibody is also referred to as a "scFv". The scFv is a molecule in which one light chain variable region and one heavy chain variable region are linked via a peptide linker. The scFv has one antigen binding site. The segment encoding the scFv is a part of region of the nucleic acid molecule of the present embodiment. In the nucleic acid molecule of Figure 1, the segment encoding the antigen binding region includes a nucleotide sequence encoding an scFv composed of VH, L and VL. In Fig. 1, the nucleotide sequence encoding the scFv includes, but is not limited to, a segment encoding each of VH, L, and VL in order from the 5' side. The nucleotide sequence encoding the scFv may include a segment encoding each of VL, L, and VH in order from the 5' side. Details of each segment of the nucleic acid molecule of the present embodiment and the CAR encoded by the nucleic acid molecule are described later.

Hereinafter, the FR3 of the light chain variable region is also referred to as a "light chain FR3". As exemplified in Fig. 1, the nucleic acid molecule of the present embodiment is characterized in that at least three codons in the nucleotide sequence encoding a light chain FR3 defined by the Chothia method are codons encoding acidic amino acid residues. However, the at least three codons include at least three codons selected from the group consisting of a codon encoding an amino acid residue at position 60, a codon encoding an amino acid residue at position 63, a codon encoding an amino acid residue at position 65, a codon encoding an amino acid residue at position 67, a codon encoding an amino acid residue at position 70, a codon encoding an amino acid residue at position 72, a codon encoding an amino acid residue at position 74, and a codon encoding an amino acid residue at position 76 of the light chain variable region. As long as at least three of the codons encoding the amino acid residues at these positions are codons encoding acidic amino acid residues, codons encoding amino acid residues at other positions of the light chain FR3 may be codons encoding any of acidic amino acid residues, basic amino acid residues, and neutral amino acid residues. As used herein, the term "codon" refers to three consecutive nucleotides of DNA or RNA. The Chothia method is described later.

Therefore, the CAR encoded by the nucleic acid molecule of the present embodiment has an antigen binding region in which at least three amino acid residues of the light chain FR3 defined by the Chothia method are acidic amino acid residues, and the at least three amino acid residues include at least three selected from the group consisting of an amino acid residue at position 60, an amino acid residue at position 63, an amino acid residue at position 65, an amino acid residue at position 67, an amino acid residue at position 70, an amino acid residue at position 72, an amino acid residue at position 74, and an amino acid residue at position 76 of the light chain variable region. Such antigen binding regions are hereinafter also referred to as "modified antigen binding regions". A CAR having a modified antigen binding region is hereinafter also referred to as a "post-modification CAR". The nucleic acid molecule of the present embodiment can be said to be a nucleic acid molecule encoding the post-modification CAR.

The framework region (FR) is a region other than the complementarity determining region (CDR) present in each of the light chain variable region and the heavy chain variable region of an antibody. The FR serves as a scaffold linking the three CDRs and contributes to the structural stability of the CDRs. Therefore, the amino acid sequence of the FR is highly conserved among antibodies of the same species. In the variable region of each of the heavy chain and the light chain, there are three CDRs of CDR1, CDR2 and CDR3 and four FRs of FR1, FR2, FR3 and FR4. These are arranged in the order of FR1, CDR1, FR2, CDR2, FR3, CDR3 and FR4 from the N-terminal side of the variable region. Hereinafter, the CDR of the heavy chain may be referred to as "HCDR", and the CDR of the light chain may be referred to as "LCDR".

In the art, the method of numbering the amino acid residue of a CDR (hereinafter, also referred to as "numbering method") to define the boundary and length of the CDR is known. When the amino acid residue of the CDR is numbered by the numbering method, the amino acid residue of the FR is also numbered. The number numbered to the amino acid residue by the numbering method indicates the position of the amino acid residue in the amino acid sequence of the light chain or the heavy chain. Examples of the numbering method include the Chothia method (Chothia C. and Lesk AM., Canonical Structures for the Hypervariable Regions of Immunoglobulins., J Mol Biol., vol. 196, p. 901-917, 1987), the Kabat method (Kabat EA.et al., Sequences of Proteins of Immunological Interest., NIH publication No. 91-3242), the IMGT method (Lefranc MP., et al., Developmental and Comparative Immunology 29 (2005) 185-203), the Honegger method (Honegger A. et al., Yet Another Numbering Scheme for Immunoglobulin Variable Domains: An Automatic Modeling and Analysis Tool., J Mol Biol., vol. 309, p. 657-670, 2001), the ABM method, and the Contact method. The numbering in the present specification is numbering when the first amino acid residue in the light chain variable region is designated as "position 1".

With respect to the antigen binding region, the boundaries, and lengths of CDRs and FRs in each variable region of the light chain and the heavy chain may be defined by any numbering method. In the present specification, boundaries and lengths of CDR and FR are defined by the Chothia method. For example, when the antigen binding region includes or consists of an scFv, according to the Chothia method, the light chain FR1 of the scFv is defined as an region consisting of amino acid residues at positions 1 to 23 of the light chain variable region. The light chain FR2 of an scFv is defined as a region consisting of amino acid residues at positions 35 to 49 of the light chain variable region. The light chain FR3 of scFv is defined as a region consisting of amino acid residues at positions 57 to 88 of the light chain variable region. The light chain FR4 of scFv is defined as a region consisting of amino acid residues at positions 98 to 110 of the light chain variable region. In the present specification, when the position of an amino acid residue in the light chain variable region of the antigen binding region is described, unless otherwise specified, the position of the amino acid residue represents a position defined by the Chothia method.

The nucleic acid molecule encoding the modified antigen binding region can be obtained by modification of a codon described later with respect to the nucleic acid molecule encoding the original antigen binding region. In the present specification, the "original antigen binding region" refers to an antigen binding region before being modified, in which the number of acidic amino acid residues at positions 60, 63, 65, 67, 70, 72, 74, and 76 of the light chain variable region is two or less. That is, in the nucleotide sequence encoding the light chain variable region of the original antigen binding region, there are two or less codons encoding acidic amino acid residues in the codons encoding the amino acid residues at the above positions. In the nucleotide sequence encoding the light chain variable region of the original antigen binding region, the codon encoding an amino acid residue other than the above positions may be a codon encoding any of an acidic amino acid residue, a basic amino acid residue, and a neutral amino acid residue. Hereinafter, the CAR having the original antigen binding region is also referred to as a "pre-modification CAR".

The nucleic acid molecule of the present embodiment can be obtained by linking a nucleic acid molecule encoding the extracellular domain, a nucleic acid molecule encoding the transmembrane domain, and a nucleic acid molecule encoding the intracellular domain in order from the 5' side. These nucleic acid molecules can be linked by known genetic recombination techniques and other molecular biological techniques. The nucleic acid molecule encoding the extracellular domain may consist of a nucleic acid molecule encoding a modified antigen binding region. Preferably, the nucleic acid molecule encoding the extracellular domain is obtained by linking a nucleic acid molecule encoding a modified antigen binding region and a nucleic acid molecule encoding a hinge domain by the above-described technology.

In the nucleotide sequence encoding the light chain FR3 of the original antigen binding region, changing three or more codons encoding amino acid residues that is not acidic amino acid residues into codons encoding acidic amino acid residues is hereinafter also referred to as "modify a codon" or" modification of a codon". The nucleic acid molecule encoding the modified antigen binding region can be obtained by performing modification of a codon of the nucleic acid molecule encoding the original antigen binding region. The amino acid residue that is not an acidic amino acid residue is a neutral amino acid residue and/or a basic amino acid residue, and is preferably a neutral amino acid residue. The nucleotide sequence encoding the modified antigen binding region and the nucleotide sequence encoding the original antigen binding region are preferably the same except for a portion where a codon is modified.

By such a modification of a codon, at least three codons in the nucleotide sequence encoding the light chain FR3 of the original antigen binding region become codons encoding acidic amino acid residues, and a nucleic acid molecule encoding a modified antigen binding region can be obtained. The modification of a codon can be performed by substituting or inserting a codon in a nucleic acid molecule encoding the original antigen binding region.

The acidic amino acid residue is an aspartic acid residue and a glutamic acid residue. The basic amino acid residue is a lysine residue and an arginine residue. The neutral amino acid residue is an alanine residue, an asparagine residue, a cysteine residue, a glycine residue, a glutamine residue, an isoleucine residue, a leucine residue, a methionine residue, a phenylalanine residue, a proline residue, a serine residue, a threonine residue, a tryptophan residue, a tyrosine residue, a valine residue, and a histidine residue. The histidine residue is treated herein as a neutral amino acid residue, as they may exhibit weak basicity or neutrality in a neutral environment. In particular, the histidine residue is treated as a neutral amino acid residue in the calculation of the electrical characteristic (X) of CDR described later.

In the nucleotide sequence encoding the light chain FR3, at least three codons introduced by modification of a codon may be all codons encoding an aspartic acid residue or all codons encoding a glutamic acid residue. Alternatively, in the nucleotide sequence encoding the light chain FR3, at least three codons introduced by modification of a codon may be codons a part of which encodes an aspartic acid residue and the rest of which encodes a glutamic acid residue.

The nucleic acid molecule of the present embodiment may be DNA or RNA. In the nucleotide sequence encoding the light chain FR3, the type of codon introduced by modification of a codon is not particularly limited as long as it encodes an acidic amino acid residue. When the nucleic acid molecule of the present embodiment is DNA, examples of the codon encoding an acidic amino acid residue include GAC and GAT encoding an aspartic acid residue, and GAA and GAG encoding a glutamic acid residue. When the nucleic acid molecule of the present embodiment is RNA, examples of the codon encoding an acidic amino acid residue include GAC and GAU encoding an aspartic acid residue, and GAA and GAG encoding a glutamic acid residue.

In the nucleic acid molecule of the present embodiment, the number of codons encoding acidic amino acid residues in the nucleotide sequence encoding the light chain FR3 is, for example, 3, 4, 5, 6, 7, 8, 9, 10, 11, 12, 13, or 14. The number of codons encoding acidic amino acid residues in the nucleotide sequence encoding the light chain FR3 is preferably 3 or more and 6 or less, and more preferably 3 or more and 5 or less.

In the light chain FR3 of the modified antigen binding region, it is preferable that at least three acidic amino acid residues derived from the modification of a codon are at the positions of amino acid residues obtained by removing the vernier zone residue and the unexposed residue from the amino acid sequence of the light chain FR3. The "vernier zone residue" is an amino acid residue that contributes to the structural stability of CDR in the amino acid sequence of FR. The "unexposed residue" is an amino acid residue that is folded inside the molecule and is not exposed to the surface. It is expected that even if the unexposed residues are modified, the effect of the modification is small or absent. For example, the amino acid residues obtained by removing the vernier zone residues and the unexposed residues from the amino acid sequence of the light chain FR3 are amino acid residues at positions 57, 58, 59, 60, 61, 62, 63, 65, 67, 70, 72, 74, 76, 77, 79, 80, and 81 of the light chain variable region. Therefore, in the nucleotide sequence encoding the modified antigen binding region, when a codon encoding amino acid residues other than positions 60, 63, 65, 67, 70, 72, 74, and 76 of the light chain variable region is modified, the modified codon is preferably a codon encoding amino acid residues at positions selected from the group consisting of positions 57, 58, 59, 61, 62, 77, 79, 80, and 81.

As described above, in the nucleotide sequence encoding the modified antigen binding region, at least three codons selected from the group consisting of a codon encoding an amino acid residue at position 60, a codon encoding an amino acid residue at position 63, a codon encoding an amino acid residue at position 65, a codon encoding an amino acid residue at position 67, a codon encoding an amino acid residue at position 70, a codon encoding an amino acid residue at position 72, a codon encoding an amino acid residue at position 74, and a codon encoding an amino acid residue at position 76 in the light chain variable region are codons encoding an acidic amino acid residue. Preferably, three or more and six or less codons selected from the above group are codons encoding acidic amino acid residues. More preferably, three or more and five or less codons selected from the above group are codons encoding acidic amino acid residues.

For example, in the nucleotide sequence encoding the modified antigen binding region, codons encoding at least three amino acid residues selected from the group consisting of positions 63, 65, 67, 70, and 72 of the light chain variable region may be codons encoding acidic amino acid residues. Alternatively, in the nucleotide sequence encoding the modified antigen binding region, codons encoding amino acid residues at positions 60, 74, and 76 of the light chain variable region may be codons encoding acidic amino acid residues.

For example, in the nucleotide sequence encoding the modified antigen binding region, codons encoding at least two amino acid residues selected from the group consisting of positions 63, 65, 67, 70, and 72 of the light chain variable region and codons encoding at least one amino acid residue selected from the group consisting of positions 60, 74, and 76 of the light chain variable region may be codons encoding acidic amino acid residues. Alternatively, in the nucleotide sequence encoding the modified antigen binding region, a codon encoding at least one amino acid residue selected from the group consisting of positions 63, 65, 67, 70, and 72 of the light chain variable region and a codon encoding at least two amino acid residues selected from the group consisting of positions 60, 74, and 76 of the light chain variable region may be codons encoding an acidic amino acid residue.

In the nucleotide sequence encoding the light chain FR3 of the modified antigen binding region, when three or more and six or less codons are codons encoding acidic amino acid residues, the three or more and six or less codons may include, for example, codons encoding three, four, or five amino acid residues selected from the group consisting of positions 63, 65, 67, 70, and 72 of the light chain variable region, and codons encoding one, two, or three amino acid residues selected from the group consisting of positions 60, 74, and 76 of the light chain variable region. Alternatively, the three or more and six or less codons may include, for example, codons encoding one, two, or three amino acid residues selected from the group consisting of positions 63, 65, 67, 70, and 72 of the light chain variable region, and codons encoding two or three amino acid residues selected from the group consisting of positions 60, 74, and 76 of the light chain variable region.

In the nucleotide sequence encoding the light chain FR3 of the modified antigen binding region, when three or more and five or less codons are codons encoding acidic amino acid residues, the three or more and five or less codons may include, for example, codons encoding two or three amino acid residues selected from the group consisting of positions 63, 65, 67, 70, and 72 of the light chain variable region, and codons encoding one or two amino acid residues selected from the group consisting of positions 60, 74, and 76 of the light chain variable region. Alternatively, the three or more and five or less codons may include, for example, codons encoding one or two amino acid residues selected from the group consisting of positions 63, 65, 67, 70, and 72 of the light chain variable region, and codons encoding two or three amino acid residues selected from the group consisting of positions 60, 74, and 76 of the light chain variable region.

In the nucleotide sequence encoding the light chain FR3 of the modified antigen binding region, for example, each codon according to any one of the following 1) to 11) may be a codon encoding an acidic amino acid residue.
1) A codon encoding an amino acid residue at position 60, a codon encoding an amino acid residue at position 63, and a codon encoding an amino acid residue at position 65 in the light chain variable region;
2) A codon encoding the amino acid residue at position 60, a codon encoding the amino acid residue at position 74, and a codon encoding the amino acid residue at position 76 in the light chain variable region;
3) A codon encoding the amino acid residue at position 63, a codon encoding the amino acid residue at position 70, and a codon encoding the amino acid residue at position 72 in the light chain variable region;
4) A codon encoding the amino acid residue at position 67, a codon encoding the amino acid residue at position 70, and a codon encoding the amino acid residue at position 72 in the light chain variable region;
5) A codon encoding the amino acid residue at position 70, a codon encoding the amino acid residue at position 72, and a codon encoding the amino acid residue at position 74 in the light chain variable region;
6) A codon encoding the amino acid residue at position 63, a codon encoding the amino acid residue at position 65, a codon encoding the amino acid residue at position 67, and a codon encoding the amino acid residue at position 70 in the light chain variable region;
7) A codon encoding the amino acid residue at position 63, a codon encoding the amino acid residue at position 65, a codon encoding the amino acid residue at position 67, and a codon encoding the amino acid residue at position 72 in the light chain variable region;
8) A codon encoding the amino acid residue at position 63, a codon encoding the amino acid residue at position 65, a codon encoding the amino acid residue at position 70, and a codon encoding the amino acid residue at position 72 in the light chain variable region;
9) A codon encoding the amino acid residue at position 63, a codon encoding the amino acid residue at position 67, a codon encoding the amino acid residue at position 70, and a codon encoding the amino acid residue at position 72 in the light chain variable region;
10) A codon encoding the amino acid residue at position 65, a codon encoding the amino acid residue at position 67, a codon encoding the amino acid residue at position 70, and a codon encoding the amino acid residue at position 72 in the light chain variable region; and
11) A codon encoding the amino acid residue at position 63, a codon encoding the amino acid residue at position 65, a codon encoding the amino acid residue at position 67, a codon encoding the amino acid residue at position 70, and a codon encoding the amino acid residue at position 72 in the light chain variable region.

Since the CDR is involved in the affinity of the antigen binding region for the antigen, it is preferable that the nucleotide sequence encoding the CDR of the antigen binding region is not changed in the nucleic acid molecule of the present embodiment. That is, the amino acid sequence of the CDR of the modified antigen binding region and the nucleotide sequence encoding the same are preferably the same as the amino acid sequence of the CDR of the original antigen binding region and the nucleotide sequence encoding the same.

The electrical characteristic of the CDR of the antigen binding region encoded by the nucleic acid molecule of the present embodiment is not particularly limited, and may be any of negative charge, neutral, and positive charge. The same applies to the original antigen binding region. The electrical characteristic of CDR is an index determined based on the number of acidic amino acid residues and basic amino acid residues of CDR included in one antigen binding site. The CDR included in one antigen binding site is all CDRs present in one heavy chain variable region and one light chain variable region constituting one antigen binding site. That is, the CDR included in one antigen binding site is a total of six CDRs of LCDR1, LCDR2, LCDR3, HCDR1, HCDR2, and HCDR3. The electrical characteristic of CDR is specifically determined by the following formula (I). When the antigen binding region includes a plurality of antigen binding sites, the electrical characteristic of CDR can be determined for each antigen binding site.X = [Number of basic amino acid residues in CDR included in one antigen binding region] - [Number of acidic amino acid residues in CDR included in one antigen binding region] ...
wherein when X is -2 or less, the electrical characteristic of CDR is negatively charged,
when X is -1, 0, or 1, the electrical characteristic of CDR is neutral, and
when X is 2 or more, the electrical characteristic of CDR is positively charged.

As can be seen from the above formula (I), the electrical characteristic of the CDR of the antigen binding region is determined by the difference between the total number of basic amino acid residues included in the six CDRs of LCDR1, LCDR2, LCDR3, HCDR1, HCDR2, and HCDR3 in the antigen binding region and the total number of acidic amino acid residues. In the above formula, the acidic amino acid residues are an aspartic acid residue and a glutamic acid residue, and the basic amino acid residues are an arginine residue and a lysine residue. As described above, the histidine residue is treated as a neutral amino acid residue in the calculation of the electrical characteristic of CDR. For example, in the scFv that binds to the MAGE-A4/HLA-A2 complex of Example 1 described later, when CDR is defined by the Chothia method, the number of acidic amino acid residues and the number of basic amino acid residues in a CDR included in one antigen binding site are 6 and 3, respectively (see Table 1). Therefore, the electrical characteristic of CDR of the scFv is negative charge.

Hereinafter, the immune cell including the pre-modification CAR is also referred to as a "pre-modification immune cell", and the immune cell including the post-modification CAR is also referred to as a "post-modification immune cell". When the amino acid sequence of the post-modification CAR is the same as the amino acid sequence of the pre-modification CAR except that at least three amino acid residues of the light chain FR3 are acidic amino acid residues, the post-modification immune cell and the pre-modification immune cell can be compared for cytotoxic activity. The term "cytotoxic activity" is synonymous with "cytocidal effect". In the post-modification immune cell, the cytotoxic activity against a tumor cell is improved as compared with the pre-modification immune cell.

The cytotoxic activity of an immune cell including the CAR can be evaluated in vitro. For example, the cytotoxic activity can be evaluated by an index such as the survival number or survival rate of a tumor cell when the immune cell including the CAR and the tumor cell are co-cultured. Specifically, the method of Example 1 described below is illustrated. The cytotoxic activity of the immune cell including the CAR can also be evaluated in vivo using a non-human animal. For example, when solid cancer is targeted, the cytotoxic activity can be evaluated by transplanting the tumor cell into a non-human animal such as a mouse, then administering the immune cell including the CAR, and measuring the size of the tumor. In the case of using a blood cancer as a subject, the cytotoxic activity can be evaluated by transplanting the tumor cell into a non-human animal such as a mouse, then administering the immune cell including the CAR, and measuring the number of tumor cells in the collected blood with a microscope, a flow cytometer, or the like.

As described above, the nucleic acid molecule encoding the modified antigen binding region can be obtained from the nucleic acid molecule encoding the original antigen binding region by known genetic recombination techniques and other molecular biological techniques. First, a primer set for modifying a codon is produced based on the nucleotide sequence of the nucleic acid molecule encoding the original antigen binding region. For example, when the modification of a codon is substitution of a codon, a primer set designed so that at least three codons in the nucleotide sequence encoding the light chain FR3 are substituted with codons encoding acidic amino acid residues is produced. Then, by a PCR method using this primer set, a nucleic acid molecule encoding the original antigen binding region is amplified as a template, whereby a nucleic acid molecule encoding an antigen binding region in which at least three amino acid residues of the light chain FR3 are substituted with acidic amino acid residues can be obtained. Alternatively, when the modification of a codon is insertion of a codon, a primer set designed to insert a codon encoding an acidic amino acid residue at at least three positions in the nucleotide sequence encoding the light chain FR3 is produced. Then, by a PCR method using this primer set, a nucleic acid molecule encoding the original antigen binding region is amplified as a template, whereby a nucleic acid molecule encoding the antigen binding region in which acidic amino acid residues are inserted at at least three positions of the light chain FR3 can be obtained.

When having a nucleic acid molecule encoding a pre-modification CAR, a nucleic acid molecule encoding a CAR including a modified antigen binding region can be produced by In-Fusion (registered trademark) cloning as in Example 1 described later. For example, first, a DNA fragment is synthesized in which at least three codons in the nucleotide sequence encoding the light chain FR3 are codons encoding acidic amino acid residues. Next, from the plasmid DNA vector including the gene encoding a pre-modification CAR, a linearized vector in which a portion corresponding to the fragment is deleted is prepared by an inverse PCR method. Then, the DNA fragment and the linearized vector are ligated with an In-Fusion reagent. This results in a nucleic acid molecule encoding a CAR including a modified antigen binding region.

As described above, since the nucleic acid molecule encoding the modified antigen binding region is obtained based on the nucleic acid molecule encoding the original antigen binding region, it is preferable that the original antigen binding region has available a nucleic acid molecule including a nucleotide sequence encoding the original antigen binding region. For example, when an E. coli clone having a plasmid DNA encoding the original antigen binding region is available, a nucleic acid molecule including a nucleotide sequence encoding the original antigen binding region can be obtained by extracting the plasmid DNA from the E. coli clone. In order to produce the primer set, it is preferable that the nucleotide sequence encoding the original antigen binding region is known or can be confirmed. When the antigen binding region includes or consists of an scFv, the nucleotide sequence encoding the scFv can be examined from known databases such as, for example, PDB, GeneBank, abYsis, and IMGT. When a nucleic acid molecule including a nucleotide sequence encoding the original antigen binding region is possessed, the nucleotide sequence encoding the original antigen binding region can be examined by sequencing the nucleic acid molecule.

The modified antigen binding region and the original antigen binding region preferably bind to at least an antigen expressed in a tumor cell. The antigen may be an antigen that is also expressed in a normal cell or an antigen that is specifically expressed in a tumor cell. The antigen includes not only a full-length antigen but also a fragment of the antigen and a complex of the fragment of the antigen and a MHC (major histocompatibility gene complex) protein. Examples of the fragment of the antigen include a part of the antigen presented by the antigen-presenting cell or the tumor cell itself, a synthetic peptide composed of a part of the amino acid sequence of the antigen, and the like. The MHC protein is called HLA (human leukocyte antigen) in humans, and includes HLA-A, HLA-B, HLA-C, HLA-DR, HLA-DQ, HAL-DP, and the like. For example, when the antigen is a protein expressed in a tumor cell, the modified antigen binding region and the original antigen binding region can bind to a complex of a fragment of the antigen and an MHC protein.

As the type of antigen recognized by the antigen binding region, an antigen presents on the surface of a tumor cell and an antigen presents in a tumor cell are preferable. Examples of such an antigen include CD19, CD20, CD30, CD44, CD133, MAGE-A1, MAGE-A2, MAGE-A3, MAGE-A4, PRAME, NY-ESO-1, SSX2, GP100, MART-1, HER2, carcinoembryonic antigen (CEA), MUC-1, CA125, glypican 3 (GPC3), B cell maturation antigen (BCMA), prostate-specific membrane antigen (PSMA), ganglioside GM2, mesothelin, and the like. The antigen recognized by the antigen binding region may be a complex of a fragment of the antigen and an MHC protein. This complex is formed when an antigen is fragmented in a tumor cell and the fragment is bound to MHC of the tumor cell. Then, the formed complex is presented on the surface of the tumor cell. Examples of the complex include a complex of a MAGE-A4 derived peptide and HLA-A2 (hereinafter also referred to as "MAGE-A4/HLA-A2 complex"), and a complex of a PRAME derived peptide and HLA-A24 (hereinafter also referred to as "PRAME/HLA-A24 complex"). Examples of the HLA-A2 forming a complex with the MAGE-A4 derived peptide include HLA-A*02:01, HLA-A*02:02, HLA-A*02:03, HLA-A*02:05, HLA-A*02:06, HLA-A*02:07, HLA-A*02:11, and the like. Among them, HLA-A*02:01 is preferable. Examples of the HLA-A24 forming a complex with a PRAME derived peptide include HLA-A*24:02 and HLA-A*24:03. Among them, HLA-A*24:02 is preferable. The MAGE-A4 derived peptide and the PRAME derived peptide may form a complex with HLA different from the above depending on the sequence of the peptide to be fragmented. The peptide derived from MAGE-A4 derived peptide and the PRAME derived peptide are oligopeptides consisting of a part (for example, 8 to 20 amino acids) of amino acid sequence of MAGE-A4 and PRAME, respectively. Examples of the MAGE-A4 derived peptide include oligopeptides composed of the amino acid sequence of GVYDGREHTV (SEQ ID NO: 1). Examples of the FRAME derived peptide include oligopeptides composed of an amino acid sequence of LYVDSLFFL (SEQ ID NO: 2).

The nucleic acid molecule itself encoding the original antigen binding region can be obtained by known genetic recombination techniques and other molecular biological techniques. When the antigen binding region includes or consists of an scFv, for example, a nucleic acid molecule encoding an scFv that binds to an antigen of a tumor cell can be isolated by phage display using an antibody phage library. Alternatively, a hybridoma that produces an antibody that binds to an antigen of a tumor cell can be produced, and a nucleic acid molecule encoding the scFv can be produced by a reverse transcription reaction and a PCR method using RNA extracted from the hybridoma. The hybridoma can be produced by a known method such as the method described in Kohler G. and Milstein C., Nature, vol. 256, p. 495-497, 1975.

In the segment encoding the extracellular domain (hereinafter, also referred to as "extracellular segment"), the nucleotide sequence encoding the antigen binding region includes a nucleotide sequence encoding a light chain variable region and a nucleotide sequence encoding a heavy chain variable region. The nucleotide sequence encoding the antigen binding region may include a nucleotide sequence encoding a part or all of the constant region of the antibody. The constant region of the antibody may be either the constant region of the heavy chain or the constant region of the light chain, but is preferably the constant region of the light chain. In the nucleotide sequence encoding the antigen binding region, the order of the nucleotide sequence encoding the light chain variable region and the nucleotide sequence encoding the heavy chain variable region is not particularly limited. For example, the nucleotide sequence encoding the antigen binding region may include, in order from the 5' side, a nucleotide sequence encoding a light chain variable region and a nucleotide sequence encoding a heavy chain variable region. Alternatively, the nucleotide sequence encoding the antigen binding region may include, in order from the 5' side, a nucleotide sequence encoding the heavy chain variable region and a nucleotide sequence encoding the light chain variable region. When a nucleotide sequence encoding a part or all of the constant region of the light chain is included, the nucleotide sequence preferably follows the nucleotide sequence encoding the light chain variable region. When a nucleotide sequence encoding a part or all of the constant region of the heavy chain is included, the nucleotide sequence preferably follows the nucleotide sequence encoding the heavy chain variable region.

The segment encoding the antigen binding region includes or consists of a nucleotide sequence encoding an scFv that binds to any one of the antigens of the tumor cell. Preferably, the segment encoding the antigen binding region includes or consists of a nucleotide sequence encoding a complex of a MAGE-A4 derived peptide and HLA-A2, an scFv that binds CD19, BCMA or CEA.

Preferably, the nucleotide sequence encoding the scFv that binds to a complex of a MAGE-A4 derived peptide and HLA-A2 includes the nucleotide sequence set forth in SEQ ID NOs: 3 to 7 and a nucleotide sequence consisting of GATGATAGC. SEQ ID NO: 3 denotes the nucleotide sequence encoding HCDR1; SEQ ID NO: 4 denotes the nucleotide sequence encoding HCDR2; SEQ ID NO: 5 denotes the nucleotide sequence encoding HCDR3; SEQ ID NO: 6 denotes the nucleotide sequence encoding LCDR1; and SEQ ID NO: 7 denotes the nucleotide sequence encoding LCDR3. The nucleotide sequence consisting of GATGATAGC encodes LCDR2. A specific example of a nucleotide sequence encoding an scFv including a nucleotide sequence consisting of SEQ ID NOs: 3 to 7 and GATGATAGC is shown in SEQ ID NO: 8. A nucleotide sequence encoding an scFv in which the light chain FR3 is modified based on the nucleotide sequence shown in SEQ ID NO: 8 is shown, for example, in SEQ ID NOs: 9 to 13.

Preferably, the nucleotide sequence encoding the scFv that binds to CD19 includes the nucleotide sequence set forth in SEQ ID NOs: 14 to 17, a nucleotide sequence consisting of CACACATCT, and a nucleotide sequence consisting of GGTAGCGAA. SEQ ID NO: 14 denotes the nucleotide sequence encoding HCDR1, SEQ ID NO: 15 denotes the nucleotide sequence encoding HCDR3, SEQ ID NO: 16 denotes the nucleotide sequence encoding LCDR1, and SEQ ID NO: 17 denotes the nucleotide sequence encoding LCDR3. The nucleotide sequence consisting of CACACATCT encodes HCDR2. The nucleotide sequence consisting of GGTAGCGAA encodes LCDR2. A specific example of a nucleotide sequence encoding an scFv including SEQ ID NOs: 14 to 17, a nucleotide sequence consisting of CACACATCT and a nucleotide sequence consisting of GGTAGCGAA is shown in SEQ ID NO: 18. A nucleotide sequence encoding an scFv in which the light chain FR3 is modified based on the nucleotide sequence shown in SEQ ID NO: 18 is shown, for example, in SEQ ID NOs: 19 to 26.

The nucleotide sequence encoding the scFv preferably includes a nucleotide sequence encoding a peptide linker between the nucleotide sequence encoding the light chain variable region and the nucleotide sequence encoding the heavy chain variable region. In scFv, a peptide linker is a moiety linking a light chain variable region and a heavy chain variable region. The amino acid sequence of the peptide linker is not particularly limited, but generally, a sequence having a length of 15 to 20 amino acids including repetition of an amino acid sequence consisting of a glycine residue and a serine residue (for example, GGGGS: SEQ ID NO: 27) is used. For example, the nucleotide sequence encoding the scFv may include, in order from the 5' side, a nucleotide sequence encoding a light chain variable region, a nucleotide sequence encoding a peptide linker, and a nucleotide sequence encoding a heavy chain variable region. Alternatively, the nucleotide sequence encoding the scFv may include, in order from the 5' side, a nucleotide sequence encoding a heavy chain variable region, a nucleotide sequence encoding a peptide linker, and a nucleotide sequence encoding a light chain variable region.

In the CAR, the transmembrane domain is a site for immobilizing the CAR on the cell membrane of the immune cell. The transmembrane domain of the CAR may be derived from a transmembrane protein. For example, in the nucleic acid molecule of the present embodiment, a segment having a nucleotide sequence encoding a transmembrane domain (hereinafter, also referred to as "transmembrane segment") may include a nucleotide sequence encoding the full length of a transmembrane protein. Alternatively, a transmembrane segment may include a nucleotide sequence encoding a part of a transmembrane protein as long as the function as a transmembrane domain is maintained. A part of the transmembrane protein preferably includes all or a part of the transmembrane region of the protein. The transmembrane protein is not particularly limited, and examples thereof include an α chain of a T cell receptor, a β chain of a T cell receptor, CD3ε, CD3ζ, CD4, CD5, CD8α, CD9, CD16, CD22, CD28, CD33, CD37, CD45, CD64, CD80, CD86, CD134, CD154, 4-1BB (also referred to as CD137), ICOS (Inducible T-cell co-stimulator) and GITR (Glucocorticoid-induced TNF receptor). Preferably, the transmembrane segment includes a nucleotide sequence encoding a transmembrane region of any one of the proteins selected from the group of these transmembrane proteins. Among them, a nucleotide sequence encoding the transmembrane region of CD8α or CD28 is particularly preferable. A specific example of the nucleotide sequence encoding the transmembrane region of CD8α is shown in SEQ ID NO: 28. A specific example of a nucleotide sequence encoding the transmembrane region of CD28 is shown in SEQ ID NO: 29.

The nucleic acid molecule of the present embodiment preferably includes a segment encoding a hinge domain (hereinafter, also referred to as "hinge segment") between the nucleotide sequence encoding the antigen binding region and the transmembrane segment. In the CAR, the hinge domain may confer a length for the antigen binding region to access the antigen and flexibility to avoid steric hindrance to the extracellular domain. The hinge domain is also referred to as a spacer region. The hinge domain of the CAR can be derived from, for example, a transmembrane protein or IgG. Specifically, in the nucleic acid molecule of the present embodiment, the hinge segment may include a nucleotide sequence encoding the entire extracellular region of the transmembrane protein or the entire constant region of IgG. Alternatively, the hinge segment may include a nucleotide sequence encoding a part of the extracellular region of a transmembrane protein or a part of the constant region of an IgG as long as the function as a hinge domain is maintained. Of the extracellular region of the membrane protein, a portion that can be used as a hinge domain is hereinafter also referred to as a "hinge region". Preferably, the hinge segment includes a nucleotide sequence encoding any one region selected from the group consisting of the constant region of the light chain of IgG, the hinge region of CD8α, and the hinge region of CD28. The IgG is preferably IgG4. A specific example of a nucleotide sequence encoding the constant region of the light chain of IgG is shown in SEQ ID NO: 30. A specific example of a nucleotide sequence encoding the CD28 hinge region is shown in SEQ ID NO: 31. A specific example of the nucleotide sequence encoding the hinge region of CD8α is shown in SEQ ID NO: 117.

In the CAR, the intracellular domain includes a signaling domain. The signaling domain of the CAR is a site for inducing signaling that activates an immune cell expressing the CAR when the antigen binding region binds to an antigen. In the nucleic acid molecule of the present embodiment, the segment encoding the intracellular domain (hereinafter, also referred to as "intracellular segment") may include a nucleotide sequence encoding a signaling domain. The signaling domain of the CAR may be derived from a membrane protein having an intracellular region, such as a cell membrane receptor, a transmembrane protein, or the like. A signaling domain may be present in the intracellular region of the membrane protein. That is, the intracellular domain of the CAR may include a signaling domain of a membrane protein. Thus, the intracellular segment may include a nucleotide sequence encoding the full length of the membrane protein. Alternatively, the intracellular segment may include a nucleotide sequence encoding a part of the membrane protein as described above as long as the function as a signaling domain is maintained. A part of the membrane protein preferably includes all or a part of the signaling domain of the protein. The membrane protein is not particularly limited, and examples thereof include CD3ζ, CD3γ, CD3δ, CD3ε, CD5, CD22, CD66d, CD79a, CD79b, FcRy, and FcRβ. Preferably, the intracellular segment includes a nucleotide sequence encoding a signaling domain of at least one protein selected from the group of these membrane proteins. Among them, CD3ζ is preferable. Specific examples of nucleotide sequences encoding the signaling domain of CD3ζ are shown in SEQ ID NOs: 32 and 118.

The intracellular segment preferably has a nucleotide sequence encoding a co-stimulatory domain in addition to the nucleotide sequence encoding the signaling domain described above. It is known that the signal from the co-stimulatory domain is transmitted to an immune cell (particularly T cell) together with the signal from the signaling domain, thereby improving the proliferation capability, cytotoxic activity, survival rate, and the like of the immune cell. The co-stimulatory domain of the CAR may be derived from a membrane protein having an intracellular region, such as a cell membrane receptor, a transmembrane protein, or the like. A co-stimulatory domain may be present in the intracellular region of the membrane protein. That is, the intracellular domain of the CAR may include a co-stimulatory domain of a membrane protein. Thus, the intracellular segment may include a nucleotide sequence encoding the full length of the membrane protein. Alternatively, the intracellular segment may include a nucleotide sequence encoding a part of the membrane proteins described above as long as the function as a co-stimulatory domain is maintained. A part of the membrane protein preferably includes all or a part of the co-stimulatory domain of the protein. The membrane protein is not particularly limited, and examples thereof include 4-1BB (CD137), CD28, GITR, CD2, CD5, CD8, CD9, CD16, CD22, CD33, CD37, CD64, CD80, CD86, CD134, CD154, and ICOS. Preferably, the intracellular segment includes a nucleotide sequence encoding a co-stimulatory domain of at least one protein selected from the group of these membrane proteins. Among them, at least one selected from the group consisting of 4-1BB, CD28, and GITR is preferable. A specific example of a nucleotide sequence encoding the co-stimulatory domain of 4-1BB is shown in SEQ ID NO: 33. A specific example of a nucleotide sequence encoding the co-stimulatory domain of CD28 is shown in SEQ ID NO: 34. A specific example of a nucleotide sequence encoding the co-stimulatory domain of GITR is shown in SEQ ID NO: 35.

In the intracellular domain segment, the order of the nucleotide sequence encoding the signaling domain and the nucleotide sequence encoding the co-stimulatory domain is not particularly limited. For example, the nucleotide sequence encoding the intracellular domain may include, in order from the 5' side, a nucleotide sequence encoding the signaling domain and a nucleotide sequence encoding the co-stimulatory domain. Alternatively, the nucleotide sequence encoding the intracellular domain may include, in order from the 5' side, a nucleotide sequence encoding the co-stimulatory domain and a nucleotide sequence encoding the signaling domain.

The nucleic acid molecule of the present embodiment may include various nucleotide sequences in addition to the nucleotide sequence encoding the CAR as necessary. Examples of such a nucleotide sequence include a leader sequence, a recognition sequence of a restriction enzyme, a nucleotide sequence encoding a peptide tag, a stop codon, and the like. The peptide tag can be appropriately selected from known peptide tags such as a histidine tag, a glutathione-S-transferase (GST) tag, and a FLAG (registered trademark) tag.

A further embodiment of the present invention relates to a vector including the nucleic acid molecule in the above section 1. Specifically, the vector of the present embodiment may be in a form in which the nucleic acid molecule of the present embodiment is incorporated into a known vector. The type of vector is not particularly limited, and examples thereof include a plasmid vector and a viral vector. The vector may be linear or circular. The type of plasmid vector is not particularly limited, and examples thereof include an expression vector, a vector for producing a viral vector, a transposon vector, and a cloning vector. An expression vector is a vector that enables the expression of a protein encoded by the nucleotide sequence of a nucleic acid molecule incorporated into the vector to be expressed in an appropriate host cell such as a mammalian cell, an insect cell, a yeast, or an E. coli. The transposon vector is a vector that enables the incorporation of the nucleic acid molecule incorporated into a transposon vector into the genome of a host cell by being introduced into an appropriate host together with an expression vector in which a gene encoding a transposase is incorporated.

The type of viral vector is not particularly limited, and examples thereof include a retroviral vector, a lentiviral vector, an adenoviral vector, an adeno-associated virus (AAV) vector, a vaccinia virus vector, and an Epstein-Barr virus (EBV) vector. The viral vector is preferably defective in replication capacity so that the virus does not self-replicate in the infected cell.

The vector may include appropriate control sequences as necessary. Examples of such a control sequence include a promoter sequence, an operator sequence, an enhancer sequence, a nucleotide sequence encoding a drug resistance marker, a multiple cloning site, and the like.

As described above, the nucleic acid molecule of the present embodiment may be either DNA or RNA. DNA is a stable substance compared to RNA, and various DNA vectors are commercially available. Therefore, the nucleic acid molecule of the present embodiment which is DNA is advantageous in that it is easy to store and handle. It is known that when RNA encoding a protein is introduced into a cell, the protein can be expressed without being affected by a transcriptional regulatory process. Therefore, the nucleic acid molecule of the present embodiment which is RNA is advantageous in that rapid expression of the CAR is enabled in the immune cell.

### 2. CAR

A further embodiment of the present invention relates to a CAR. The CAR of the present embodiment is a protein molecule encoded by the nucleotide sequence of the nucleic acid molecule described in the above section 1. That is, the CAR of the present embodiment is the above-described "post-modification CAR". The CAR of the present embodiment is a protein produced by a genetic engineering technique including an extracellular domain, a transmembrane domain, and an intracellular domain in this order from the N-terminal side. The extracellular domain includes an antigen binding region including a light chain variable region and a heavy chain variable region. The CAR of present embodiment is characterized in that at least three amino acid residues are acidic amino acid residues in the light chain FR3 of the antigen binding region. However, the at least three amino acid residues include at least three selected from the group consisting of an amino acid residue at position 60, an amino acid residue at position 63, an amino acid residue at position 65, an amino acid residue at position 67, an amino acid residue at position 70, an amino acid residue at position 72, an amino acid residue at position 74, and an amino acid residue at position 76 in the light chain variable region. As long as at least three of the amino acid residues at these positions are acidic amino acid residues, the amino acid residues at other positions of the light chain FR3 may be any of acidic amino acid residues, basic amino acid residues, and neutral amino acid residues.

As described above, the CAR of the present embodiment has an antigen binding region in which at least three amino acid residues of the light chain FR3 defined by the Chothia method are acidic amino acid residues, and the at least three amino acid residues include at least three selected from the group consisting of an amino acid residue at position 60, an amino acid residue at position 63, an amino acid residue at position 65, an amino acid residue at position 67, an amino acid residue at position 70, an amino acid residue at position 72, an amino acid residue at position 74, and an amino acid residue at position 76 of the light chain variable region. The antigen binding region is the same as the modified antigen binding region described above. That is, the CAR of the present embodiment has a modified antigen binding region. The CAR of the present embodiment can also be said to be a mutant of the CAR having the original antigen binding region. The CAR of the present embodiment is preferably expressed in an immune cell and immobilized on a cell membrane of the immune cell. In the immune cell expressing the CAR, the cytotoxic activity against a tumor cell is improved as compared with the immune cell expressing the CAR including the original antigen binding region.

In the CAR of present embodiment, at least three acidic amino acid residues of the light chain FR3 are derived from the modification of a codon. In the light chain FR3 of the modified antigen binding region, the at least three acidic amino acid residues derived from the modification of a codon may be all aspartic acid residues or all glutamic acid residues. Alternatively, in at least three acidic amino acid residues derived from modification of a codon, some of which may be aspartic acid residues and the remainder may be glutamic acid residues.

The amino acid residues before at least three amino acid residues in the light chain FR3 are converted into acidic amino acid residues is neutral amino acid residues and/or basic amino acid residues, preferably neutral amino acid residues. That is, at least three acidic amino acid residues in the light chain FR3 of the modified antigen binding region are residues changed from at least three residues selected from a neutral amino acid residue and/or a basic amino acid residue in the light chain FR3 of the original antigen binding region.

In the light chain FR3 of the modified antigen binding region, the number of at least three acidic amino acid residues derived from modification of a codon is, for example, 3, 4, 5, 6, 7, 8, 9, 10, 11, 12, 13, or 14. The number of at least three acidic amino acid residues derived from modification of a codon in the light chain FR3 of the modified antigen binding region is preferably 3 or more and 6 or less, and more preferably 3 or more and 5 or less. As described above, it is preferable that at least three acidic amino acid residues in the light chain FR3 of the modified antigen binding region are at the positions of amino acid residues obtained by removing the vernier zone residue and the unexposed residue from the amino acid sequence of the light chain FR3. Therefore, when an amino acid residue at a position other than positions 60, 63, 65, 67, 70, 72, 74, and 76 of the light chain variable region in the modified antigen binding region is an acidic amino acid residue by modification of a codon, the position is preferably any of positions 57, 58, 59, 61, 62, 77, 79, 80, and 81.

As described above, in the modified antigen binding region, at least three amino acid residues selected from the group consisting of an amino acid residue at position 60, an amino acid residue at position 63, an amino acid residue at position 65, an amino acid residue at position 67, an amino acid residue at position 70, an amino acid residue at position 72, an amino acid residue at position 74, and an amino acid residue at position 76 in the light chain variable region are acidic amino acid residues derived from modification of a codon. Preferably, three or more and six or less amino acid residues selected from the above group are acidic amino acid residues. More preferably, three or more and five or less amino acid residues selected from the above group are acidic amino acid residues.

For example, in the modified antigen binding region, at least three amino acid residues selected from the group consisting of positions 63, 65, 67, 70, and 72 of the light chain variable region may be acidic amino acid residues. Alternatively, in the modified antigen binding region, the amino acid residues at positions 60, 74 and 76 of the light chain variable region may be acidic amino acid residues.

For example, in the modified antigen binding region, at least two amino acid residues selected from the group consisting of positions 63, 65, 67, 70 and 72 of the light chain variable region and at least one amino acid residue selected from the group consisting of positions 60, 74 and 76 of the light chain variable region may be acidic amino acid residues. Alternatively, in the modified antigen binding region, at least one amino acid residue selected from the group consisting of positions 63, 65, 67, 70 and 72 of the light chain variable region and at least two amino acid residues selected from the group consisting of positions 60, 74 and 76 of the light chain variable region may be acidic amino acid residues.

In the light chain FR3 of the modified antigen binding region, when 3 to 6 amino acid residues are acidic amino acid residues, the 3 to 6 amino acid residues may include, for example, 3, 4, or 5 amino acid residues selected from the group consisting of positions 63, 65, 67, 70, and 72 of the light chain variable region and 1, 2, or 3 amino acid residues selected from the group consisting of positions 60, 74, and 76 of the light chain variable region. Alternatively, the three or more and six or less amino acid residues may include, for example, one, two, or three amino acid residues selected from the group consisting of positions 63, 65, 67, 70, and 72 of the light chain variable region, and two or three amino acid residues selected from the group consisting of positions 60, 74, and 76 of the light chain variable region.

In the light chain FR3 of the modified antigen binding region, when three or more and five or less amino acid residues are acidic amino acid residues, the three or more and five or less amino acid residues may include, for example, two or three amino acid residues selected from the group consisting of positions 63, 65, 67, 70, and 72 of the light chain variable region and one or two amino acid residues selected from the group consisting of positions 60, 74, and 76 of the light chain variable region. Alternatively, the three or more and five or less amino acid residues may include, for example, one or two amino acid residues selected from the group consisting of positions 63, 65, 67, 70, and 72 of the light chain variable region and two or three amino acid residues selected from the group consisting of positions 60, 74, and 76 of the light chain variable region.

In the light chain FR3 of the modified antigen binding region, for example, each amino acid residue described in any one of the following 1) to 11) may be an acidic amino acid residue.
1) An amino acid residue at position 60, an amino acid residue at position 63, and an amino acid residue at position 65 in the light chain variable region;
2) An amino acid residue at position 60, an amino acid residue at position 74, and an amino acid residue at position 76 in the light chain variable region;
3) A codon encoding an amino acid residue at position 63, an amino acid residue at position 70, and an amino acid residue at position 72 in the light chain variable region;
4) An amino acid residue at position 67, an amino acid residue at position 70, and an amino acid residue at position 72 in the light chain variable region;
5) An amino acid residue at position 70, an amino acid residue at position 72, and an amino acid residue at position 74 in the light chain variable region;
6) An amino acid residue at position 63, an amino acid residue at position 65, an amino acid residue at position 67, and an amino acid residue at position 70 in the light chain variable region;
7) An amino acid residue at position 63, an amino acid residue at position 65, an amino acid residue at position 67, and an amino acid residue at position 72 in the light chain variable region;
8) An amino acid residue at position 63, an amino acid residue at position 65, an amino acid residue at position 70, and an amino acid residue at position 72 in the light chain variable region;
9) An amino acid residue at position 63, an amino acid residue at position 67, an amino acid residue at position 70, and an amino acid residue at position 72 in the light chain variable region;
10) An amino acid residue at position 65, an amino acid residue at position 67, an amino acid residue at position 70, and an amino acid residue at position 72 in the light chain variable region; and
11) An amino acid residue at position 63, an amino acid residue at position 65, an amino acid residue at position 67, an amino acid residue at position 70, and an amino acid residue at position 72 in the light chain variable region.

As described above, the amino acid sequence of the CDR of the antigen binding region in the CAR of present embodiment is preferably the same as the amino acid sequence of the CDR of the original antigen binding region. The electrical characteristic of the CDR of the antigen binding region of the CAR of present embodiment is not particularly limited, and the antigen binding region may be any of negative charge, neutral, and positive charge. The electrical characteristic of CDR is determined by the above formula (I).

In the CAR of present embodiment, the extracellular domain includes an antigen binding region including a light chain variable region and a heavy chain variable region. Examples of the antigen binding region include an scFv. The antigen binding region may include a part or all of the constant regions of the antibody. The constant region of the antibody may be either the constant region of the heavy chain or the constant region of the light chain, but is preferably the constant region of the light chain. In the antigen binding region, the order of the light chain variable region and the heavy chain variable region is not particularly limited. For example, the antigen binding region may include a light chain variable region and a heavy chain variable region in order from the N-terminal side. Alternatively, the antigen binding region may include a heavy chain variable region and a light chain variable region in order from the N-terminal side. In the antigen binding region, a part or all of the constant region of the light chain is preferably included after the light chain variable region. In the antigen binding region, a part or all of the constant region of the heavy chain is preferably included after the heavy chain variable region.

In the CAR of present embodiment, the antigen binding region may include or consist of an scFv that binds to any one of the antigen present on the surface of the tumor cell and the antigen present in the tumor cell. Preferably, the antigen binding region includes or consists of a complex of a MAGE-A4 derived peptide and HLA-A2, an scFv that binds to CD 19 or CEA.

Preferably, the amino acid sequence of the scFv that binds to the complex of the MAGE-A4 derived peptide and HLA-A2 includes the amino acid sequence set forth in SEQ ID NOs: 36 to 40 and an amino acid sequence consisting of DDS (Asp-Asp-Ser). SEQ ID NO: 36 denotes the amino acid sequence of HCDR1, SEQ ID NO: 37 denotes the amino acid sequence of HCDR2, SEQ ID NO: 38 denotes the amino acid sequence of HCDR3, SEQ ID NO: 39 denotes the amino acid sequence of LCDR1, and SEQ ID NO: 40 denotes the amino acid sequence of LCDR3. The amino acid sequence composed of DDS is an amino acid sequence of LCDR2. A specific example of the amino acid sequence of the scFv including the amino acid sequence consisting of SEQ ID NO: 36 to 40 and DDS is shown in SEQ ID NO: 41. The amino acid sequence of the scFv in which the light chain FR3 is modified based on SEQ ID NO: 40 is shown in, for example, SEQ ID NOs: 42 to 46.

Preferably, the amino acid sequence of the scFv that binds to CD19 includes the amino acid sequence set forth in SEQ ID NOs: 47 to 50, an amino acid sequence consisting of GSE (Gly-Ser-Glu), and an amino acid sequence consisting of HTS (His-Thr-Ser). SEQ ID NO: 47 denotes the amino acid sequence of HCDR1, SEQ ID NO: 48 denotes the amino acid sequence of HCDR3, SEQ ID NO: 49 denotes the amino acid sequence of LCDR1, and SEQ ID NO: 50 denotes the amino acid sequence of LCDR3. The amino acid sequence composed of GSE is an amino acid sequence of HCDR2, and the amino acid sequence composed of HTS is an amino acid sequence of LCDR2. A specific example of the amino acid sequence of the scFv including SEQ ID NOs: 47 to 50 is shown in SEQ ID NO: 51. The amino acid sequence of the scFv in which the light chain FR3 is modified based on SEQ ID NO: 51 is shown in, for example, SEQ ID NOs: 52 to 59.

The scFv preferably includes a peptide linker between the light chain variable region and the heavy chain variable region. The peptide linker is as described above. For example, the scFv may include a light chain variable region, a peptide linker, and a heavy chain variable region in order from the N-terminal side. Alternatively, the scFv may include a heavy chain variable region, a peptide linker, and a light chain variable region in order from the N-terminal side.

In the CAR of present embodiment, the transmembrane domain may include the full length of the transmembrane protein. Alternatively, the transmembrane domain may include a part of a transmembrane protein as long as its function is maintained. A part of the transmembrane protein preferably includes all or a part of the transmembrane region of the protein. Examples of the transmembrane protein that can be used for the transmembrane domain include the proteins exemplified in the above section 1. Preferably, the transmembrane domain is, for example, a transmembrane region of any one protein selected from the group consisting of an α chain of a T cell receptor, a β chain of a T cell receptor, CD3ε, CD3ζ, CD4, CD5, CD8α, CD9, CD16, CD22, CD28, CD33, CD37, CD45, CD64, CD80, CD86, CD134, CD154, 4-1BB (CD137), ICOS and GITR. Among them, the transmembrane region of CD8α or CD28 is preferable. A specific example of the amino acid sequence of the transmembrane region of CD8α is shown in SEQ ID NO: 60. A specific example of the amino acid sequence of the transmembrane region of CD28 is shown in SEQ ID NO: 61.

The CAR of the present embodiment preferably includes a hinge domain between the antigen binding region and the transmembrane domain. The hinge domain may include the entire extracellular region of the transmembrane protein or the entire constant region of IgG. Alternatively, a hinge domain may include a part of the extracellular region of a transmembrane protein or a part of the constant region of an IgG as long as its function is maintained. Examples of the protein that can be used for the hinge domain include the proteins exemplified in the above section 1. Preferably, the hinge domain is, for example, any one region selected from the group consisting of a constant region of a light chain of IgG, a hinge region of CD8α, and a hinge region of CD28. The IgG is preferably IgG4. A specific example of the amino acid sequence of the constant region of the light chain of IgG is shown in SEQ ID NO: 62. A specific example of the amino acid sequence of the CD28 hinge region is shown in SEQ ID NO: 63. A specific example of the amino acid sequence of the hinge region of CD8α is shown in SEQ ID NO: 119.

In the CAR of present embodiment, the intracellular domain may include the full length of a membrane protein having an intracellular region. Alternatively, the intracellular domain may include a part of the membrane protein described above as long as the function as a signaling domain is maintained. The signaling domain may be present in the intracellular region of the membrane protein. A part of the membrane protein having the intracellular region preferably includes all or a part of the signaling domain of the protein. Examples of the membrane protein that can be used for the intracellular domain include the proteins exemplified in the above section 1. Preferably, the intracellular domain includes, for example, a signaling domain of at least one protein selected from the group consisting of CD3ζ, CD3γ, CD3δ, CD3ε, CD5, CD22, CD66d, CD79a, CD79b, FcRγ and FcRβ. Among them, the signaling domain of CD3ζ is preferred. Specific examples of the amino acid sequence of the signaling domain of CD3ζ are shown in SEQ ID NOs: 64 and 120.

In the CAR of present embodiment, the intracellular domain preferably has a co-stimulatory domain in addition to the signaling domain. The intracellular domain may include the full length of a membrane protein having an intracellular region. Alternatively, the intracellular domain may include a part of a membrane protein as described above as long as the function as a co-stimulatory domain is maintained. The co-stimulatory domain may be present in the intracellular region of each of the above proteins. A part of the membrane protein having an intracellular region preferably includes all or a part of the co-stimulatory domain of the protein. Examples of the membrane protein that can be used for the co-stimulatory domain include the proteins exemplified in the above section 1. Preferably, the co-stimulatory domain includes a co-stimulatory domain of at least one protein selected from the group consisting of, for example, 4-1BB (CD137), CD28, GITR, CD2, CD5, CD8, CD9, CD16, CD22, CD33, CD37, CD64, CD80, CD86, CD134, CD154 and ICOS. Among them, co-stimulatory domains of 4-1BB, CD28 and GITR are preferred. A specific example of the amino acid sequence of the co-stimulatory domain of 4-1BB is shown in SEQ ID NO: 65. A specific example of the amino acid sequence of the co-stimulatory domain of CD28 is shown in SEQ ID NO: 66. A specific example of the amino acid sequence of the co-stimulatory domain of GITR is shown in SEQ ID NO: 67.

In the intracellular domain, the order of the signaling domain and the co-stimulatory domain is not particularly limited. For example, the intracellular domain may include a signaling domain and a co-stimulatory domain in order from the N-terminal side. Alternatively, the intracellular domain may include a co-stimulatory domain and a signaling domain in order from the N-terminal side.

The CAR of the present embodiment may include an additional oligopeptide or polypeptide as necessary. Examples of such a oligopeptide and a polypeptide include a signal peptide, a peptide tag, and the like. The peptide tag can be appropriately selected from known peptide tags such as a histidine tag, a GST tag, and a FLAG (registered trademark) tag.

The CAR of the present embodiment can be produced by a protein expression system using the nucleic acid molecule of the present embodiment. The protein expression system may be an expression system using a host cell or a cell-free protein synthesis system. In the generation by an expression system using a host cell, for example, the CAR of the present embodiment can be expressed by introducing the nucleic acid molecule of the present embodiment incorporated into an expression vector suitable for the host cell to be used into the host cell. Examples of the cell-free protein synthesis system include a wheat germ derived synthesis system, an E. coli derived synthesis system, and a reconfigurable cell-free protein synthesis system. When the CAR is produced in the host cell, the host cell may be lysed with a solution including a suitable solubilizing agent to release the CAR in the solution. In the cell-free protein synthesis system, the synthesized CAR is included in the reaction solution. The CAR released in the liquid can be recovered by a known method such as column chromatography. For example, when the produced CAR has a histidine tag or a GST tag as a peptide tag, the peptide can be recovered by affinity chromatography using a carrier including Ni-NTA (nitrilotriacetic acid that forms a chelate with a nickel ion) or glutathione. The recovered CAR may be purified by a known method such as gel filtration or dialysis, as necessary.

### 3. Immune cell including CAR

A further embodiment of the invention relates to an immune cell including the CAR. The immune cell including the CAR of present embodiment is an immune cell expressing the CAR in the above section 2. by introducing the nucleic acid molecule of the above section 1. into the immune cell. The details of the CAR and the nucleic acid molecule are as described above. Examples of the immune cell before the nucleic acid molecule described in the above section 1. is introduced include immune cells collected from mammals including humans, immune cells prepared from stem cells, and immune cells established as cell lines. Specifically, immune cells obtained by leukapheresis, immune cells isolated from blood, and the like are exemplified.

Examples of the immune cell separated or isolated from the biological sample include peripheral blood mononuclear cells (PBMC), T cells, and natural killer (NK) cells. Among them, T cells and NK cells are preferred. T cells include CD8 positive T cells, CD4 positive T cells, cytotoxic T cells, helper T cells, regulatory T cells, and tumor-infiltrating lymphocytes. Preferred T cells are CD8 positive T cells and cytotoxic T cells. The cell line of immune cells is preferably a cell line derived from lymphocytes, and examples thereof include Jurkat cells, MOLT-4 cells, and U-937 cells. Hereinafter, the T cell expressing the CAR may be referred to as a "CAR-T cell".

The method for introducing the nucleic acid molecule described in the above section 1. into an immune cell is not particularly limited, and can be appropriately selected from known gene introduction methods. Examples of the gene introduction method include gene introduction by a lipofection method, an electroporation method, a calcium phosphate method, a cationic polymer, gene introduction by a viral vector, and gene introduction by a transposon. In gene introduction by the lipofection method and a cationic polymer, commercially available transfection reagents such as FuGENE (registered trademark) and JetPEI (registered trademark) may be used.

The immune cell including the CAR of present embodiment can be cultured in the same manner as the immune cell before the nucleic acid molecule of present embodiment is introduced. The culture medium, serum, additive, and the like can be appropriately determined according to the type of immune cell to be used. Examples of the culture medium include MEM, DMEM, and RPMI-1640. When the immune cell is a lymphocyte, for example, a commercially available lymphocyte medium such as GT-T502, GT-T503, or GT-T551 (Takara Bio Inc.) may be used. Examples of the serum include a fetal bovine serum (FBS) and a human type AB serum. Examples of the additive include L-glutamine, insulin, and IL-2. Examples of the culture conditions for an immune cell include conditions under a 5% CO₂ atmosphere at 37°C.

In the immune cell including the CAR of the present embodiment, the CAR is expressed as a transmembrane protein. When the extracellular domain of the CAR binds to an antigen of a tumor cell, the immune cell is activated by a signal from the intracellular domain of the CAR. Then, the immune cell including the activated CAR of present embodiment exerts a cytotoxic activity by release of a cytotoxic protein (for example, perforin and granzyme) and an antitumor cytokine (for example, tumor necrosis factor (TNF)-α, lymphokine) and expression of a cell surface molecule that induces cell death such as a Fas ligand. The immune cell including the CAR of the present embodiment has improved cytotoxic activity as compared with the immune cell including the CAR having the original antigen binding region. The cytocidal effect by the immune cell including the CAR can be examined by a known method such as a cytotoxicity assay. Specifically, the method of Example 1 described below is illustrated. The cytotoxic activity can also be evaluated by administering the immune cell including the CAR to a non-human animal transplanted with a tumor cell and measuring the size of the tumor.

### 4. Method for producing immune cell including car

A further embodiment of the present invention relates to a method for producing an immune cell including the CAR (hereinafter, also referred to as "production method of the present embodiment"). In the production method of the present embodiment, the immune cell including the CAR in the above section 3. are produced by introducing the nucleic acid molecule in the above section 1. into immune cells and expressing the CAR in the above section 2. in the immune cell. The details of the CAR and the nucleic acid molecule are as described above. The details of the immune cell before the nucleic acid molecule of the above section 1. is introduced are as described above. PBMCs, T cells and NK cells are preferred. When the immune cell including the CAR obtained by the production method of the present embodiment is transplanted into a living body, it is preferable to use an immune cell separated or isolated from a biological sample collected from the living body itself or another living body of the same kind as the living body.

The method for introducing the nucleic acid molecule of the above section 1. into an immune cell is as described above. After the nucleic acid molecule is introduced into the immune cell, the immune cell is preferably cultured for a predetermined period. The method for culturing the immune cell is as described above. The predetermined period of time can be at least a period of time until the CAR is expressed in the immune cell. The period until the CAR is expressed is determined depending on the method for introducing a nucleic acid molecule, and is, for example, 3 hours or more and 72 hours or less, preferably 6 hours or more and 48 hours or less. When the method for introducing the nucleic acid molecule is a method for enabling stable expression of the CAR in the immune cell, the predetermined period may be a period sufficient for the immune cell expressing the CAR to proliferate. Such a period is not particularly limited, but is, for example, 48 hours or more and 20 days or less, preferably 72 hours or more and 14 days or less.

By introducing the nucleic acid molecule of the above section 1. into an immune cell, the CAR in the above section 2. is expressed in the immune cell. It may be confirmed that the CAR is expressed in the immune cell as necessary. The expression of the CAR in the immune cell may be confirmed by a known protein detection method such as enzyme-linked immunosorbent assay (ELISA), flow cytometry, immunoprecipitation, polyacrylamide gel electrophoresis, or Western blotting.

### 5. Pharmaceutical composition

A further embodiment of the present invention relates to a pharmaceutical composition for treating a malignant tumor including the immune cell including the CAR (hereinafter, also referred to as "pharmaceutical composition of the present embodiment"). The pharmaceutical composition of the present embodiment includes the immune cell including the CAR of the above section 3. as an active ingredient. Details of the CAR and the immune cell including the CAR are as described above. The pharmaceutical composition of the present embodiment may further include a pharmaceutically acceptable additive. Examples of such additives include aqueous media for stably preserving immune cells, D-glucose, dextran, serum albumin, and dimethyl sulfoxide (DMSO). Examples of the aqueous medium include physiological saline, phosphate buffered saline (PBS), and a composite electrolyte solution. The pharmaceutical composition of the present embodiment is preferably administered parenterally to a patient. That is, the pharmaceutical composition is preferably in a form suitable for parenteral administration, for example, in the form of an injection, and an infusion solution.

The malignant tumor to be treated by the pharmaceutical composition of the present embodiment is a tumor including a tumor cell having an antigen recognized by the CAR. The malignant tumor may be a blood cancer or a solid cancer. Examples thereof include acute leukemia (acute myeloid leukemia, B-cell acute lymphoblastic leukemia, acute monocytic leukemia, acute erythroleukemia, acute megakaryoblastic leukemia, etc.), chronic leukemia (chronic myelogenous leukemia, chronic lymphocytic leukemia, chronic monocytic leukemia, etc.), lymphoma (diffuse large B-cell lymphoma, follicular lymphoma, mantle cell lymphoma, Burkitt's lymphoma, Hodgkin's lymphoma, lymphoplasmacytic lymphoma, marginal zone lymphoma, etc.), multiple myeloma, melanoma, breast cancer, prostate cancer, bladder cancer, uterine cancer, liver cancer, kidney cancer, pancreatic cancer, spleen cancer, lung cancer, gastric cancer, esophageal cancer, ovarian cancer, osteosarcoma, and neuroblastoma.

### 6. Treatment method

A further embodiment of the present invention relates to a method for treating a malignant tumor using the immune cell including the CAR of the above section 3. (hereinafter, also referred to as "treatment method of the present embodiment"). administering the pharmaceutical composition described above to a malignant tumor patient. The malignant tumors to be treated are as described above.

In the administration step, preferably, the immune cell is administered parenterally to the patient. Parenteral administration includes, for example, intravenous, intraarterial, intramuscular, intraperitoneal, and subcutaneous administration. Among them, intravenous administration is preferable.

The administration of the pharmaceutical composition of the present embodiment can be appropriately determined according to the type of cancer, the disease state of the patient, the age, the weight, and the like. For example, for an adult with a body weight of 50 kg or more, the dose is 1×10⁴ cells or more and 1×10¹⁰ cells or less, preferably 1×10⁵ cells or more and 1×10⁹ cells or less, and more preferably 1×10⁶ cells or more and 5×10⁸ cells or less as the immune cell including the CAR per administration. The number of times of administration may be administration only once or may be administration a plurality of times. After administration, the number of administrations is usually one because the immune cell including the CAR engrafts and proliferates in the body of the patient. However, when it is determined that engraftment or proliferation of the immune cell is insufficient, the administration may be performed a plurality of times. The administration interval may be, for example, 1 to 4 times per day, every 1 week, every 10 to 30 days, every 1 month, every 3 to 6 months, or every 1 year.

The pharmaceutical composition of the present embodiment can be used in combination with other anticancer agents. The administration of the other anticancer agent and the administration of the pharmaceutical composition of the above section 5. may be performed on the same day or on different days. Examples of the other anticancer agent include, but are not limited to, alkylating agents such as cyclophosphamide, antimetabolites such as pentostatin, molecular target drugs such as rituximab, kinase inhibitors such as imatinib, proteasome inhibitors such as bortezomib, calcineurin inhibitors such as cyclosporine, anticancer antibiotics such as idarubicin, plant alkaloids such as irinotecan, platinum preparations such as cisplatin, hormone therapy agents such as tamoxifen, and immunomodulatory agents such as nivolumab and pembrolizumab.

The number of white blood cells of the patient may be reduced by performing lymphocyte-depleting chemotherapy as treatment before administration of the pharmaceutical composition of the present embodiment. For example, fludarabine, cyclophosphamide, bendamustine, and the like are used for lymphocyte-depleting chemotherapy.

### 7. Method for improving cytotoxic activity of immune cell

A further embodiment of the invention relates to a method of enhancing the cytotoxic activity of an immune cell including the CAR (hereinafter, also referred to as a "method for improving the present embodiment"). The method for improving the present embodiment includes a step of introducing the nucleic acid molecule of the above section 1. into an immune cell and expressing the CAR in the above section 2. in the immune cell. The immune cell including the obtained CAR expresses a CAR having a modified antigen binding region. The immune cell expressing the CAR has enhanced cytotoxic activity as compared with the immune cell expressing the CAR having the original antigen binding region. The improvement method of the present embodiment can contribute to more killing of tumor cells in a method for treating with the immune cell expressing a CAR. Details of the nucleic acid molecule and the CAR are as described above. The method for introducing a nucleic acid molecule into an immune cell and the method for measuring the cytotoxic activity of an immune cell including the CAR are as described above.

Hereinafter, the present invention is described in detail with reference to Examples, but the present invention is not limited to these Examples.

### EXAMPLES

### Example 1: Production of CAR-T cells binding to MAGE-A4/HLA-A2 complex and confirmation of cytotoxic activity

### (1) Acquisition of nucleic acid molecule encoding CAR as template

Plasmid DNA for production of a viral vector including a gene encoding a CAR having an scFv binding to a MAGE-A4/HLA-A2 complex was obtained in the same manner as in the Example of US2020/0276237, which is incorporated herein by reference. The above scFv binding complex was a complex of MAGE-A4 and HLA-A*02:01. Hereinafter, the CAR encoded by the gene in the plasmid DNA is referred to as "MAGE-A4-zG". The amino acid sequence of the MAGE-A4 derived peptide in the MAGE-A4/HLA-A2 complex was GVYDGREHTV (SEQ ID NO: 1). The above plasmid DNA was used as a template for producing a nucleic acid molecule encoding a MAGE-A4-zG mutant by a PCR method.

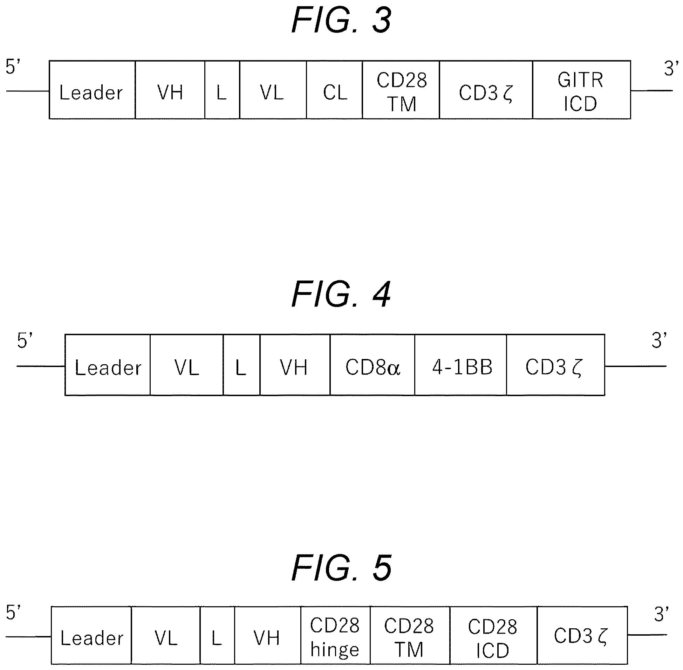

Referring to Fig. 3, in the gene encoding MAGE-A4-zG, from the 5' side, a leader sequence (Leader), a nucleotide sequence encoding a heavy chain variable region (VH), a nucleotide sequence encoding a linker (L), a nucleotide sequence encoding a light chain variable region (VL), a nucleotide sequence encoding a light chain constant region (CL), a nucleotide sequence encoding a transmembrane domain of CD28 (CD28TM), a nucleotide sequence encoding CD3ζ, and a nucleotide sequence encoding an intracellular domain of GITR (GITRICD) were linked in this order. The VH, L, VL and CL constituted a segment having a nucleotide sequence encoding the extracellular domain of the CAR. The VH, L and VL constituted a segment with a nucleotide sequence encoding an scFv that specifically binds to the MAGE-A4/HLA-A2 complex. CD3ζ and GITRICD constituted a segment with a nucleotide sequence encoding the intracellular domain of the CAR. CL was a segment with a nucleotide sequence encoding the hinge domain of the CAR. That is, MAGE-A4-zG was a CAR including, as extracellular domains, an scFv composed of VH, VL and a linker connecting them, and CL, including CD28TM as a transmembrane domain, and including CD3ζ and GITRICD as an intracellular domain.

The amino acid sequences of the CDRs of the scFv of MAGE-A4-zG are shown in Table 1. These CDRs were defined by the Chothia method using the Discovery Studio. As can be seen from Table 1, in the amino acid sequences of all CDRs, the number of acidic amino acid residues was 6, and the number of basic amino acid residues was 3. From the above formula (I), the electrical characteristic of the CDR of the scFv of MAGE-A4-zG was negatively charged (X = -3).

**[Table 1]**

| | CDR1 | CDR2 | CDR3 |
|---|---|---|---|
| Heavy chain | GGTFSSY (SEQ ID NO. 36) | PIFG (SEQ ID NO. 37) | PRRAYHDAFD (SEQ ID NO. 38) |
| Light chain | GDHIGSKS (SEQ ID NO. 39) | DDS | WDSSTA (SEQ ID NO. 40) |

### (2) Obtainment of nucleic acid molecule encoding MAGE-A4-zG mutant

### [Reagents]

- Ligation reagent: In-Fusion (registered trademark) HD Cloning Kit (Takara Bio Inc.)
- PCR reagents: PrimeSTAR (registered trademark) Max Premix (Takara Bio Inc.)
- Competent cell: ECOS (trademark) Competent E. coli DH5α (NIPPON GENE CO., LTD.)

### [Primer set]

- Forward primer: GATGAGGCTGACTATTACTGTCTG (SEQ ID NO: 68)
- Reverse primer: CCGGTCGCTATCATCATAGACGACC (SEQ ID NO: 69)

In Example 1, a nucleic acid molecule encoding three MAGE-A4-zG mutants shown in the following 1) to 3) was obtained. These mutants had an scFv in which four amino acid residues in the light chain FR3 of MAGE-A4-zG were substituted with glutamic acid residues. A specific preparation procedure is described later. Hereinafter, these mutants are referred to as "MAGE-A4-zG-Em1", "MAGE-A4-zG-Em2" and "MAGE-A4-zG-Em3", respectively. In the following 1) to 3), the amino acid residue numbers indicate positions in the VL as defined by the Chothia method.
1) MAGE-A4-zG-Em1: The amino acid residues at positions 63, 65, 67 and 70 of the VL were substituted with Glu (E) residues
2) MAGE-A4-zG-Em2: The amino acid residues at positions 63, 65, 67 and 72 of the VL were substituted with Glu (E) residues
3) MAGE-A4-zG-Em3: The amino acid residues at positions 65, 67, 70 and 72 of the VL were substituted with Glu (E) residues

Synthesis of a 114 nucleotide DNA fragment (SEQ ID NOs: 70 to 72) including codons encoding the amino acid residues shown in 1) to 3) above was entrusted to Eurofins Genomics. Using the plasmid DNA obtained in the above (1) as a template, PCR was performed using a PCR reagent and a primer set to prepare a linearized vector. This linearized vector had a sequence in which a part of the nucleotide sequence encoding the scFv of MAGE-A4-zG was deleted. The obtained linearized vector and each synthesized DNA fragment were bound by a ligation reagent. A competent cell was transformed using a ligation solution. The resulting E. coli was cultured to extract plasmid DNA. Thus, plasmid DNA for producing a viral vector including a gene encoding the MAGE-A4-zG mutant was obtained.

### (3) Preparation of immune cell

Human peripheral blood mononuclear cells (PBMC) (product number: CC2703) was purchased from Lonza Group AG. A plate for culturing PBMC was prepared as follows. Anti-CD3 antibodies OKT-3 (eBioscience) and RetroNectin (registered trademark) (Takara Bio Inc.) were added to the ACD-A solution (Terumo Corporation) at final concentrations of 5 µg/mL and 25 µg/mL, respectively. To each well of a 12 well plate (Nunc), 400 µL of the resulting solution was added, and the plate was left to stand at 4°C overnight. Each well was washed three times with PBS to obtain a PBMC culture plate. The PBMC culture medium was prepared as follows. AB serum (300 µL, Veritas) was added to the lymphocyte culture medium GT-T503(50 mL, Takara Bio Inc.), and human IL-2 (NIPRO CORPORATION) was further added so as to have a final concentration of 300 IU/mL to prepare a PBMC culture medium. PBMC was suspended in a PBMC culture medium at 2.5×10⁵ to 3.0×10⁵ cells/mL. To each well of the PBMC culture plate, 2 mL of the cell suspension was added, and cultured in a CO₂ incubator at 37°C for 3 days. On day 3, 1 mL of the medium was replaced, and the cells were cultured for another day.

### (4) Generation of immune cells expressing MAGE-A4-zG and mutants thereof

### (4.1) Generation of retrovirus by packaging cells plat-A

Plasmid DNAs for producing respective viral vectors including genes encoding MAGE-A4-zG and mutants thereof were introduced into packaging cells plat-A using FuGENE (registered trademark) (Promega K.K.) and cultured for 2 days. Thereafter, the culture supernatant was recovered to obtain a liquid including the viral vector (hereinafter, also referred to as "viral liquid").

### (4.2) Preparation of retrovirus diluent and wash solution

To a mixture of PBS (40 mL) and ACD-A solution (2.4 mL), Albuminar (registered trademark) 25% (CSL Behring K.K.) was added to a final concentration of 2.5% to prepare a retrovirus diluent. To PBS (39 mL), 25% of Albuminar (registered trademark) was added so as to have a final concentration of 1.5%, thereby preparing a retroviral washing solution.

### (4.3) Preparation of plates for retroviral infection

RetroNectin (registered trademark) (Takara Bio Inc.) was diluted with a retrovirus diluent to a final concentration of 20 µg/mL. To each well of a 24 well plate, 250 µL of a diluent of RetroNectin (registered trademark) was added and the plate was left to stand at 4°C overnight. The liquid in each well was removed and washed twice with retroviral wash. To each well, 1 mL of the viral liquid prepared in the above (4.1) was added, and centrifuged at 2000×g for 2 hours at 32°C to coat each well with retrovirus. The viral liquid in each well was removed and washed twice with a retroviral washing solution to prepare a plate for retroviral infection.

### (4.4) Introduction of CAR genes into T cells by retrovirus

The PBMCs cultured in the above (3) were recovered and suspended in a PBMC culture medium so as to be 1.3×10⁵ cells/mL. The cell suspension was added to each well of the plate for retroviral infection in an amount of 1.5 mL, and centrifuged at 1000xg for 10 minutes at 32°C. Thereafter, the plate was left to stand in a CO₂ incubator, and the cells were cultured at 37°C. Thus, T cells (CAR-T cells) expressing various CARs including MAGE-A4-zG and mutants thereof were obtained. CAR-T cells were used in various assays 11 to 14 days after PBMC separation.

### (5) Confirmation of cytotoxic activity of CAR-T cells

Using the various CAR-T cells produced in the above (4.4) as effector cells, the cytotoxic activity was measured by an N-SPC (registered trademark) non-RI cytotoxicity assay kit (TECHNO SUZUTA Co.,Ltd.). As target cells, a human melanoma cell line SK-MEL-37 was used. SK-MEL-37 was an A2 positive MAGE-A4 positive tumor cell.

The measurement principle of the above assay kit was as follows. When the BM-HT Reagent attached to the above assay kit is added to the target cells, the BM-HT Reagent is hydrolyzed by intracellular esterase to generate an HT chelate in the target cells. When the target cells are killed by effector cells, the HT chelate leaks into the culture supernatant. When the Eu Solution attached to the above assay kit is added to the culture supernatant including the HT chelate, the Eu/HT complex is formed. When the complex is excited by laser light, time-resolved fluorescence is generated. Since leakage of HT chelates depends on the cytotoxic activity of effector cells, the cytotoxic activity can be quantitatively measured by measuring time-resolved fluorescence.

The target cells were suspended in a medium so as to be 1×10⁴ cells/mL, and BM-HT Reagent was added thereto. Effector cells (CAR-T cells) and target cells were mixed at a cell number ratio of 10 : 1, added to each well of a 96 well plate, and co-cultured at 37°C for 2 hours. For comparison, two wells (control wells) to which target cells not mixed with effector cells were added were prepared and cultured in the same manner. At 1.5 hours from the start of culture, Detergent attached to the kit was added to one of the control wells, and the fluorescence detected from the control well was defined as maximum fluorescence. The other of the control wells was untreated, and the fluorescence detected from the control well was defined as minimum fluorescence. Eu Solution (120 µL) was added to the culture supernatant (12 µL) of the co-culture, and the mixture was left to stand at room temperature for 15 minutes, and then time-resolved fluorescence was measured. Measurements were performed in triplicate for each CAR-T cell.

### (6) Results

Table 2 shows the measurement results of the cytotoxic activity of each CAR-T cell. In the table, "NA" in the column of "SEQ ID NO." indicates the SEQ ID NO. of the nucleotide sequence encoding the CAR, and "AA" indicates the SEQ ID NO. of the amino acid sequence of the CAR. The column "mutation site" indicates a position at which an amino acid residue of a light chain defined by the Chothia method has been substituted with an acidic amino acid residue. "Effecter: Target" indicates the mixing ratio of the effector cells and the target cells. "Cell lysis (%)" indicates the percentage of target cells killed by each CAR-T cell. "SD" indicates the standard deviation of Cell lysis (%) of each CAR-T cell. "Ratio" indicates the ratio of Cell lysis of T cells including a MAGE-A4-zG mutant when the value of Cell lysis of T cells including MAGE-A4-zG is 1.00.

**[Table 2]**

| Name of CAR | SEQ ID NO. | | Mutation site | Effecter: Target = 10: 1 | | |
|---|---|---|---|---|---|---|
| | NA | AA | | Cell lysis (%) | SD | Ratio |
| MAGE-A4-zG | 73 | 77 | None | 33 | 7% | 1.00 |
| MAGE-A4-zG-Em1 | 74 | 78 | 63, 65, 67, 70 | 44 | 10% | 1.35 |
| MAGE-A4-zG-Em2 | 75 | 79 | 63, 65, 67, 72 | 52 | 1% | 1.56 |
| MAGE-A4-zG-Em3 | 76 | 80 | 65, 67, 70, 72 | 51 | 4% | 1.54 |

As shown in Table 2, when the mixing ratio of the effector cells and the target cells was 10 : 1, the cytotoxic activity of each mutant when the cytotoxic activity of T cells including MAGE-A4-zG was 1.00 was as follows. The cytotoxic activity of the T cells including MAGE-A4-zG-Em1 was 1.35, the cytotoxic activity of the T cells including MAGE-A4-zG-Em2 was 1.56, and the cytotoxic activity of the T cells including MAGE-A4-zG-Em3 was 1.54. For cytotoxic activity, T cells with MAGE-A4-zG mutant were higher than T cells with MAGE-A4-zG. As described above, in MAGE-A4-zG, at least three amino acid residues of the light chain FR3 defined by the Chothia method were substituted with acidic amino acid residues, whereby the cytotoxic activity of immune cells including the obtained MAGE-A4-zG mutant could be improved.

### Example 2: Confirmation of cytotoxic activity of CAR-T cells binding to MAGE-A4/HLA-A2 complex

The mixing ratio of the effector cells and the target cells was changed to 3 : 1, and the cytotoxic activity of each CAR-T cell prepared in Example 1 was measured. The target cells were SK-MEL-37. The specific measurement procedure was the same as in Example 1. The results are shown in Table 3.

**[Table 3]**

| Name of CAR | SEQ ID NO. | | Mutation site | Effecter: Target = 3: 1 | | |
|---|---|---|---|---|---|---|
| | NA | AA | | Cell lysis (%) | SD | Ratio |
| MAGE-A4-zG | 73 | 77 | None | 13 | 7% | 1.00 |
| MAGE-A4-zG-Em1 | 74 | 78 | 63, 65, 67, 70 | 33 | 10% | 2.54 |
| MAGE-A4-zG-Em2 | 75 | 79 | 63, 65, 67, 72 | 30 | 1% | 2.31 |
| MAGE-A4-zG-Em3 | 76 | 80 | 65, 67, 70, 72 | 24 | 4% | 1.85 |

As shown in Table 3, when the mixing ratio of the effector cells and the target cells was 3:1, the cytotoxic activity of each mutant when the cytotoxic activity of T cells including MAGE-A4-zG was 1.00 was as follows. The cytotoxic activity of the T cells including MAGE-A4-zG-Em1 was 2.54, the cytotoxic activity of the T cells including MAGE-A4-zG-Em2 was 2.31, and the cytotoxic activity of the T cells including MAGE-A4-zG-Em3 was 1.85. Regarding the cytotoxic activity, even when the mixing ratio between the effector cells and the target cells was changed to 3 : 1, the T cells including the MAGE-A4-zG mutant were higher than T cells including MAGE-A4-zG.

### Example 3: Production of CAR-T cells binding to MAGE-A4/HLA-A2 complex and confirmation of cytotoxic activity

### (1) Obtainment of nucleic acid molecule encoding MAGE-A4-zG mutant

In Example 3, a nucleic acid molecule encoding two MAGE-A4-zG mutants shown in the following 4) and 5) was obtained. These mutants had scFv in which three amino acid residues in the light chain FR3 of MAGE-A4-zG were substituted with glutamic acid residues or aspartic acid residues. A specific preparation procedure is described later. Hereinafter, these mutants are referred to as "MAGE-A4-zG-Em4" and "MAGE-A4-zG-Dm5", respectively. In the following 4) and 5), the amino acid residue numbers indicate positions in the VL as defined by the Chothia method.

4) MAGE-A4-zG-Em4: The amino acid residues at positions 63,70 and 72 of the VL were substituted with Glu (E) residues

5) MAGE-A4-zG-Dm5: The amino acid residues at positions 60, 74 and 76 of the VL were substituted with Asp (D) residues

The synthesis of 114 nucleotide DNA fragments (SEQ ID NOs: 81 and 82) including codons encoding the amino acid residues shown in 4) and 5) was entrusted to Eurofins Genomics. Except that this DNA fragment was used, in the same manner as in Example 1, plasmid DNA for producing a viral vector including a gene encoding a MAGE-A4-zG mutant was obtained. A viral liquid including a gene encoding MAGE-A4-zG and mutants thereof was prepared in the same manner as in Example 1.

### (2) Generation of immune cells expressing MAGE-A4-zG and mutants thereof and confirmation of activity

In the same manner as in Example 1, a gene encoding MAGE-A4-zG and mutants thereof was introduced into PBMC (Lonza) using the viral liquid to obtain each CAR-T cell. The cytotoxic activity of each CAR-T cell was measured in the same manner as in Example 1. The target cells were SK-MEL-37. The mixing ratio of the effector cells to the target cells was 3:1. The results are shown in Table 4.

**[Table 4]**

| Name of CAR | SEQ ID NO. | | Mutation site | Effecter: Target = 3: 1 | | |
|---|---|---|---|---|---|---|
| | NA | AA | | Cell lysis (%) | SD | Ratio |
| MAGE-A4-zG | 73 | 77 | None | 43 | 6% | 1.00 |
| MAGE-A4-zG-Em4 | 83 | 85 | 63, 67, 70 | 98 | 5% | 2.28 |
| MAGE-A4-zG-Dm5 | 84 | 86 | 60, 74, 76 | 84 | 4% | 1.95 |

As shown in Table 4, when the mixing ratio of the effector cells and the target cells was 3:1, the cytotoxic activity of each mutant when the cytotoxic activity of T cells including MAGE-A4-zG was 1.00 was as follows. The cytotoxic activity of the T-cells including MAGE-A4-zG-Em4 was 2.28 and the cytotoxic activity of the T-cells including MAGE-A4-zG-Dm5 was 1.95. For cytotoxic activity, the T cells including MAGE-A4-zG-Em4 or MAGE-A4-zG-Dm5 were higher than the T cells including MAGE-A4-zG. As described above, it was shown that the cytotoxic activity of immune cells including a MAGE-A4-zG mutant different from that in Example 1 was also improved.

### Example 4: Confirmation of cytotoxic activity of CAR-T cells binding to MAGE-A4/HLA-A2 complex

The mixing ratio of the effector cells and the target cells was changed to 1 : 1, and the cytotoxic activity of each CAR-T cell prepared in Example 3 was measured. The target cells were SK-MEL-37. The specific measurement procedure was the same as in Example 1. The results are shown in Table 5.

**[Table 5]**

| Name of CAR | SEQ ID NO. | | Mutation site | Effecter : Target = 1 : 1 | | |
|---|---|---|---|---|---|---|
| | NA | AA | | Cell lysis (%) | SD | Ratio |
| MAGE-A4-zG | 73 | 77 | None | 18 | 3% | 1.00 |
| MAGE-A4-zG-Em4 | 83 | 85 | 63, 67, 70 | 36 | 8% | 2.00 |
| MAGE-A4-zG-Dm5 | 84 | 86 | 60, 74, 76 | 34 | 5% | 1.89 |

As shown in Table 5, when the mixing ratio of the effector cells and the target cells was 1 : 1, the cytotoxic activity of each mutant when the cytotoxic activity of T cells including MAGE-A4-zG was 1.00 was as follows. The cytotoxic activity of the T-cells including MAGE-A4-zG-Em4 was 2.00 and the cytotoxic activity of the T-cells including MAGE-A4-zG-Dm5 was 1.89. Regarding the cytotoxic activity, even when the mixing ratio between the effector cells and the target cells was changed to 1 : 1, the T cells including MAGE-A4-zG-Em4 or MAGE-A4-zG-Dm5 were higher than the T cells including MAGE-A4-zG.

### Example 5: Generation of CAR-T cells binding to CD19 and confirmation of cytotoxic activity

### (1) Acquisition of nucleic acid molecule encoding CAR as template

By gene synthesis, plasmid DNA for producing a viral vector including a gene encoding a CAR having an scFv that binds to CD19 was obtained. Hereinafter, the CAR encoded by the gene in the plasmid DNA is referred to as "CD19-BBz". In order to produce a nucleic acid molecule encoding a CD19-BBz mutant by a PCR method, the above plasmid DNA was used as a template.

Referring to Fig. 4, in the gene encoding CD19-BBz, the leader sequence, the nucleotide sequence encoding VL, the nucleotide sequence encoding L, the nucleotide sequence encoding VH, the nucleotide sequence encoding CD8α, the nucleotide sequence encoding 4-1BB, and the nucleotide sequence encoding CD3ζ were linked in this order from the 5' side. CD19-BBz was a CAR including, as extracellular domains, an scFv composed of VH, VL and a linker linking them, including CD8α as a transmembrane domain and CD3ζ as an intracellular domain 4-1BB.

The amino acid sequences of CDRs of scFv of CD19-BBz are shown in Table 6. These CDRs were defined by the Chothia method using the Discovery Studio. As can be seen from Table 6, in the amino acid sequences of all the CDRs, the number of acidic amino acid residues was 4, and the number of basic amino acid residues was 1. From the above formula (I), the electrical characteristic of CDR of scFv of CD19-BBz was negatively charged (X = -3).

**[Table 6]**

| | CDR1 | CDR2 | CDR3 |
|---|---|---|---|
| Heavy chain | GVSLPDY (SEQ ID NO. 47) | GSE | YYYGGSYAMD (SEQ ID NO. 48) |
| Light chain | SQDISKY (SEQ ID NO. 49) | HTS | GNTLPY (SEQ ID NO. 50) |

### (2) Obtainment of a nucleic acid molecule encoding a CD19-BBz mutant

### [Primer set]

- Forward primer: GATATAGCCACCTATTTCTGCCAG (SEQ ID NO: 87)
- Reverse primer: TCCACTATGCAGCCGAGATGTGTG (SEQ ID NO: 88)

In Example 5, a nucleic acid molecule encoding two CD19-BBz mutants shown in the following 6) and 7) was obtained. These mutants had scFv in which 3 or 4 amino acid residues in the light chain FR3 of CD19-BBz were substituted with glutamic acid residues or aspartic acid residues. A specific preparation procedure is described later. Hereinafter, these mutants are referred to as "CD 19-BBz-Em6" and "CD19-BBz-Dm7", respectively. In the following 6) and 7), the amino acid residue numbers indicate positions in the VL as defined by the Chothia method.

6) CD19-BBz-Em6: The amino acid residues at positions 67, 70 and 72 of the VL were substituted with Glu (E) residues

7) CD19-BBz-Dm7: The amino acid residues at positions 63, 65, 67 and 70 of the VL were substituted with Asp (D) residues

The synthesis of 102 nucleotide DNA fragments (SEQ ID NOs: 89 and 90) including codons encoding the amino acid residues shown in 6) and 7) was entrusted to Eurofins Genomics. Using the plasmid DNA obtained in the above (1) as a template, PCR was performed using a PCR reagent and a primer set to prepare a linearized vector. This linearized vector had a sequence in which a part of the nucleotide sequence encoding the scFv of CD19-BBz was deleted. The obtained linearized vector and each synthesized DNA fragment were bound by a ligation reagent. A competent cell was transformed using a ligation solution. The resulting E. coli was cultured to extract plasmid DNA. Thus, plasmid DNA for producing a viral vector including a gene encoding the CD19-BBz mutant was obtained. The various reagents and competent cells used in Example 5 were the same as those in Example 1.

### (3) Generation of immune cells expressing CD19-BBz and mutants thereof and confirmation of activity

A viral liquid including a gene encoding CD19-BBz and mutants thereof was prepared in the same manner as in Example 1. Then, using these viral liquids, a gene encoding CD19-BBz and mutants thereof was introduced into PBMC (Lonza) to obtain each CAR-T cell. The cytotoxic activity of each CAR-T cell was measured in the same manner as in Example 1. As target cells, a human B precursor cell leukemia cell line Nalm-6 was used. Nalm-6 was CD19 positive tumor cells. The mixing ratio of the effector cells and the target cells was 1 : 1. The results are shown in Table 7.

**[Table 7]**

| Name of CAR | SEQ ID NO. | | Mutation site | Effecter: Target = 1 : 1 | | |
|---|---|---|---|---|---|---|
| | NA | AA | | Cell lysis (%) | SD | Ratio |
| CD19-BBz | 91 | 94 | None | 11 | 3% | 1.00 |
| CD19-BBz-Em6 | 92 | 95 | 67, 70, 72 | 34 | 4% | 3.09 |
| CD19-BBz-Dm7 | 93 | 96 | 63, 65, 67, 70 | 22 | 2% | 2.00 |

As shown in Table 7, when the mixing ratio of the effector cells and the target cells was 1 : 1, the cytotoxic activity of each mutant when the cytotoxic activity of T cells including CD19-BBz was 1.00 was as follows. Cytotoxic activity of the T cells including CD19-BBz-Em6 was 3.09 and cytotoxic activity of the T cells including CD19-BBz-Dm7 was 2.00. As described above, in CD19-BBz, by substituting at least 3 amino acid residues of the light chain FR3 defined by the Chothia method with acidic amino acid residues, the cytotoxic activity of immune cells including the obtained CD19-BBz mutant could be improved.

### Example 6: Generation of CAR-T cells binding to CD19 and confirmation of cytotoxic activity

### (1) Acquisition of nucleic acid molecule encoding CAR as template

By gene synthesis, plasmid DNA for producing a viral vector including a gene encoding a CAR having an scFv that binds to CD19 was obtained. Hereinafter, the CAR encoded by the gene in the plasmid DNA is referred to as "CD19-28z". The above plasmid DNA was used as a template for producing a nucleic acid molecule encoding a CD19-28z mutant by a PCR method.

Referring to Fig. 5, in the gene encoding CD19-28z, from the 5' side, a leader sequence, a nucleotide sequence encoding VL, a nucleotide sequence encoding L, a nucleotide sequence encoding VH, a nucleotide sequence encoding a hinge domain (CD28 hinge) of CD28, a nucleotide sequence encoding CD28TM, a nucleotide sequence encoding an intracellular domain (CD28ICD) of CD28, and a nucleotide sequence encoding CD3ζ were linked in this order. CD19-28z was a CAR including, as extracellular domains, an scFv consisting of VH, VL and a linker connecting them, and a hinge domain of CD28, including CD28TM as a transmembrane domain, and including CD28ICD and CD3ζ as intracellular domains. The scFv of CD19-28z was the same as the scFv of CD19-BBz in Example 5. The amino acid sequences of CDRs of scFv of CD19-28z were as shown in Table 6.

### (2) Obtainment of a nucleic acid molecule encoding a CD19-28z mutant

In Example 6, a nucleic acid molecule encoding six CD19-28z mutants shown in the following 8) to 13) was obtained. These mutants had scFv in which 3, 4 or 5 amino acid residues in the light chain FR3 of CD19-28z were substituted with aspartic acid residues or glutamic acid residues. A specific preparation procedure is described later. Hereinafter, these mutants are referred to as "CD19-28z-Dm8", "CD19-28z-Em9", "CD19-28z-Dm10", "CD19-28z-Dm11", "CD19-28z-Dm12" and "CD19-28z-Em13", respectively. In the following 8) to 13), the amino acid residue numbers indicate positions in the VL as defined by the Chothia method.
8) CD19-28z-Dm8: The amino acid residues at positions 60, 63 and 65 of the VL were substituted with Asp (D) residues
9) CD19-28z-Em9: The amino acid residues at positions 70, 72 and 74 of the VL were substituted with Glu (E) residues
10) CD19-28z-Dm10: The amino acid residues at positions 63, 65, 70 and 72 of the VL were substituted with Asp (D) residues
11) CD19-28z-Dm11: The amino acid residues at positions 63, 67, 70 and 72 of the VL were substituted with Asp (D) residues
12) CD19-28z-Dm12: The amino acid residues at positions 63, 65, 67, 70 and 72 of the VL were substituted with Asp (D) residues
13) CD19-28z-Em13: The amino acid residues at positions 63, 65, 67, 70 and 72 of the VL were substituted with Glu (E) residues

The synthesis of a 102 nucleotide DNA fragment (SEQ ID NOs: 97 to 102) including codons encoding the amino acid residues shown in 8) to 13) above was entrusted to Eurofins Genomics. Using the plasmid DNA obtained in the above (1) as a template, PCR was performed using a PCR reagent and a primer set to prepare a linearized vector. This linearized vector had a sequence in which a part of the nucleotide sequence encoding the scFv of CD19-28z was deleted. The obtained linearized vector and each synthesized DNA fragment were bound by a ligation reagent. A competent cell was transformed using a ligation solution. The resulting E. coli was cultured to extract plasmid DNA. Thus, plasmid DNA for producing a viral vector including a gene encoding the CD19-28z mutant was obtained. The various reagents and competent cells used in Example 6 were the same as those in Example 1. The primer set used in Example 6 was the same as that used in Example 5.

### (3) Generation of immune cells expressing CD19-28z and mutants thereof and confirmation of activity

A viral liquid including a gene encoding CD19-28z and mutants thereof was prepared in the same manner as in Example 1. Then, using these viral liquids, a gene encoding CD19-28z and mutants thereof was introduced into PBMC (Lonza) to obtain each CAR-T cell. The cytotoxic activity of each CAR-T cell was measured in the same manner as in Example 1. The target cells were Nalm-6. The mixing ratio of the effector cells to the target cells was 3:1. The results are shown in Table 8.

**[Table 8]**

| Name of CAR | SEQ ID NO. | | Mutation site | Effecter: Target = 3: 1 | | |
|---|---|---|---|---|---|---|
| | NA | AA | | Cell lysis (%) | SD | Ratio |
| CD19-28z | 103 | 110 | None | 50 | 6% | 1.00 |
| CD19-28z-Dm8 | 104 | 111 | 60, 63, 65 | 60 | 5% | 1.20 |
| CD 1 9-28z-Em9 | 105 | 112 | 70, 72, 74 | 94 | 7% | 1.88 |
| CD19-28z-Dm10 | 106 | 113 | 63, 65, 70, 72 | 98 | 6% | 1.96 |
| CD19-28z-Dm11 | 107 | 114 | 63, 67, 70, 70 | 81 | 21% | 1.62 |
| CD19-28z-Dm12 | 108 | 115 | 63, 65, 67, 70, 72 | 75 | 9% | 1.50 |
| CD19-28z-Em13 | 109 | 116 | 63, 65, 67, 70, 72 | 68 | 11% | 1.36 |

As shown in Table 8, when the mixing ratio of the effector cells to the target cells was 3 : 1, the cytotoxic activity of each mutant when the cytotoxic activity of T cells including CD19-28z was taken as 1.00 was as follows. The cytotoxic activity of the T cells including CD19-28z-Dm8 was 1.20, the cytotoxic activity of the T cells including CD19-28z-Em9 was 1.88, the cytotoxic activity of the T cells including CD 19-28z-Dm 10 was 1.96, the cytotoxic activity of the T cells including CD19-28z-Dm11 was 1.62, the cytotoxic activity of the T cells including CD19-28z-Dm12 was 1.50, and the cytotoxic activity of the T cells including CD19-28z-Em13 was 1.36. As described above, in CD19-28z, by substituting at least 3 amino acid residues of the light chain FR3 defined by the Chothia method with acidic amino acid residues, the cytotoxic activity of immune cells including the obtained CD19-28z mutant could be improved.

### Example 7: Confirmation of cytotoxic activity of CAR-T cells binding to CD19

The mixing ratio of the effector cells and the target cells was changed to 1 : 1, and the cytotoxic activity of each CAR-T cell prepared in Example 6 was measured. The target cells were Nalm-6. The specific measurement procedure was the same as in Example 1. The results are shown in Table 9.

**[Table 9]**

| Name of CAR | SEQ ID NO. | | Mutation site | Effecter: Target = 1 : 1 | | |
|---|---|---|---|---|---|---|
| | NA | AA | | Cell lysis (%) | SD | Ratio |
| CD19-28z | 103 | 110 | None | 21 | 9% | 1.00 |
| CD19-28z-Dm8 | 104 | 111 | 60, 63, 65 | 26 | 12% | 1.24 |
| CD19-28z-Em9 | 105 | 112 | 70, 72, 74 | 39 | 25% | 1.86 |
| CD19-28z-Dm10 | 106 | 113 | 63, 65, 70, 72 | 50 | 3% | 2.38 |
| CD19-28z-Dm11 | 107 | 114 | 63, 67, 70, 70 | 49 | 5% | 2.33 |
| CD19-28z-Dm12 | 108 | 115 | 63, 65, 67, 70, 72 | 47 | 9% | 2.24 |
| CD19-28z-Em13 | 109 | 116 | 63, 65, 67, 70, 72 | 32 | 17% | 1.52 |

As shown in Table 9, when the mixing ratio of the effector cells and the target cells was 1 : 1, the cytotoxic activity of each mutant when the cytotoxic activity of T cells including CD19-28z was 1.00 was as follows. The cytotoxic activity of the T cells including CD19-28z-Dm8 was 1.24, the cytotoxic activity of T cells including CD19-28z-Em9 was 1.86, the cytotoxic activity of T cells including CD19-28z-Dm10 was 2.38, the cytotoxic activity of T cells including CD19-28z-Dm11 was 2.33, the cytotoxic activity of T cells including CD19-28z-Dm12 was 2.24, and the cytotoxic activity of T cells including CD19-28z-Em13 was 1.52. Regarding the cytotoxic activity, even when the mixing ratio between the effector cells and the target cells was changed to 1 : 1, T cells including the CD19-28z mutant were higher than the T cells including CD19-28z.

## Claims

1. A nucleic acid molecule comprising a nucleotide sequence encoding a chimeric antigen receptor, wherein
the nucleic acid molecule comprises a segment encoding an extracellular domain, a segment encoding a transmembrane domain, and a segment encoding an intracellular domain,
the segment encoding the extracellular domain comprises a nucleotide sequence encoding an antigen binding region comprising a light chain variable region and a heavy chain variable region,
at least three codons in a nucleotide sequence encoding framework region 3 of the light chain variable region as defined by the Chothia method are codons encoding acidic amino acid residues, and
the at least three codons comprise at least three selected from the group consisting of a codon encoding an amino acid residue at position 60, a codon encoding an amino acid residue at position 63, a codon encoding an amino acid residue at position 65, a codon encoding an amino acid residue at position 67, a codon encoding an amino acid residue at position 70, a codon encoding an amino acid residue at position 72, a codon encoding an amino acid residue at position 74, and a codon encoding an amino acid residue at position 76 in the light chain variable region.

2. The nucleic acid molecule according to claim 1, wherein 3 or more and 5 or less codons selected from the group consisting of a codon encoding an amino acid residue at position 60, a codon encoding an amino acid residue at position 63, a codon encoding an amino acid residue at position 65, a codon encoding an amino acid residue at position 67, a codon encoding an amino acid residue at position 70, a codon encoding an amino acid residue at position 72, a codon encoding an amino acid residue at position 74, and a codon encoding an amino acid residue at position 76 in the light chain variable region are codons encoding an acidic amino acid residue.

3. The nucleic acid molecule according to claim 1, wherein the antigen binding region comprises a single-chain antibody, and the single-chain antibody is a single-chain antibody that binds to a complex of a MAGE-A4 derived peptide and HLA-A2, CD19, BCMA, or CEA.

4. The nucleic acid molecule according to claim 1, wherein the transmembrane domain comprises a transmembrane region of any one protein selected from the group consisting of an α chain of a T cell receptor, a β chain of a T cell receptor, CD3ε, CD3ζ, CD4, CD5, CD8, CD9, CD16, CD22, CD28, CD33, CD37, CD45, CD64, CD80, CD86, CD134, CD154, 4-1BB, ICOS, and GITR.

5. The nucleic acid molecule according to claim 1, wherein the intracellular domain comprises a signaling domain of at least one protein selected from the group consisting of CD3ζ, CD3γ, CD3δ, CD3ε, CD5, CD22, CD66d, CD79a, CD79b, FcRy, and FcRβ.

6. The nucleic acid molecule of claim 5, wherein
the segment encoding the intracellular domain further comprises a nucleotide sequence encoding a co-stimulatory domain, and
the co-stimulatory domain is a co-stimulatory domain of at least one protein selected from the group consisting of 4-1BB, CD28, GITR, CD2, CD5, CD8, CD9, CD16, CD22, CD33, CD37, CD64, CD80, CD86, CD134, CD154 and ICOS.

7. The nucleic acid molecule according to claim 1, further comprising a segment encoding a hinge domain between the nucleotide sequence encoding the antigen binding region and the segment encoding the transmembrane domain.

8. The nucleic acid molecule according to claim 1, wherein the nucleic acid molecule is DNA or RNA.

9. A vector comprising the nucleic acid molecule of any one of claim 1 to 8.

10. An immune cell comprising a chimeric antigen receptor comprising an extracellular domain, a transmembrane domain and an intracellular domain, wherein
the extracellular domain comprises an antigen binding region comprising a light chain variable region and a heavy chain variable region,
at least three amino acid residues of framework region 3 of the light chain variable region as defined by the Chothia method are acidic amino acid residues, and
the at least three amino acid residues comprises at least three selected from the group consisting of an amino acid residue at position 60, an amino acid residue at position 63, an amino acid residue at position 65, an amino acid residue at position 67, an amino acid residue at position 70, an amino acid residue at position 72, an amino acid residue at position 74, and an amino acid residue at position 76 in the light chain variable region.

11. A pharmaceutical composition comprising the immune cell of claim 10.

12. A pharmaceutical composition for treating a malignant tumor comprising the immune cell of claim 10.

13. A method for improving cytotoxic activity of an immune cell comprising a chimeric antigen receptor,
wherein the chimeric antigen receptor comprises an extracellular domain, a transmembrane domain and an intracellular domain, and the extracellular domain comprises an antigen binding region comprising a light chain variable region and a heavy chain variable region,
the method comprising
changing at least three amino acid residues of framework region 3 of the light chain variable region defined by the Chothia method to acidic amino acid residues to improve cytotoxic activity of an immune cell including the chimeric antigen receptor as compared to a chimeric antigen receptor before changing the at least three amino acid residues to acidic amino acid residues.

14. A method for producing an immune cell comprising a chimeric antigen receptor, comprising introducing the nucleic acid molecule according to any one of claim 1 to 8 into an immune cell, and expressing the chimeric antigen receptor in the immune cell.

15. A method for producing an immune cell comprising a chimeric antigen receptor, comprising introducing the vector according to claim 9 into an immune cell, and expressing the chimeric antigen receptor in the immune cell.
